(19) Europäisches Patentamt
European Patent Office
Office européen des brevets

(11) EP 4 326 223 B1

(12) # EUROPÄISCHE PATENTSCHRIFT

(45) Veröffentlichungstag und Bekanntmachung des Hinweises auf die Patenterteilung:
**06.08.2025 Patentblatt 2025/32**

(21) Anmeldenummer: **22718667.3**

(22) Anmeldetag: **28.03.2022**

(51) Internationale Patentklassifikation (IPC):
***A61K 8/34** (2006.01)* ***A61K 8/86** (2006.01)*
***A61K 8/898** (2006.01)* ***A61Q 5/06** (2006.01)*
***A61Q 5/10** (2006.01)*

(52) Gemeinsame Patentklassifikation (CPC):
**A61K 8/345; A61K 8/86; A61K 8/898; A61Q 5/065; A61Q 5/10;** A61K 2800/31; A61K 2800/43

(86) Internationale Anmeldenummer:
**PCT/EP2022/058059**

(87) Internationale Veröffentlichungsnummer:
**WO 2022/223238 (27.10.2022 Gazette 2022/43)**

(54) **WASSERFREIES MITTEL ZUM FÄRBEN VON KERATINISCHEM MATERIAL, INSBESONDERE MENSCHLICHEN HAAREN, ENTHALTEND AMINOSILIKONE, PIGMENTE UND BESTIMMTE ALKYLENGLYCOLE**

ANHYDROUS AGENT FOR DYEING KERATINOUS MATERIAL, IN PARTICULAR HUMAN HAIR, CONTAINING AMINO SILICONES, PIGMENTS AND PARTICULAR ALKYLENE GLYCOLS

PRODUIT ANHYDRE POUR LA COLORATION D’UNE MATIÈRE KÉRATINIQUE, EN PARTICULIER DES CHEVEUX, CONTENANT DES AMINOSILICONES, DES PIGMENTS ET DES ALKYLÈNEGLYCOLS DÉFINIS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.04.2021 DE 102021203907**

(43) Veröffentlichungstag der Anmeldung:
**28.02.2024 Patentblatt 2024/09**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**

(56) Entgegenhaltungen:
**WO-A1-2015/185069 WO-A1-2020/260097**

- **DATABASE GNPD [online] MINTEL; 19 September 2018 (2018-09-19), ANONYMOUS: "Powder Foundation Long Keep SP Refill", XP055951389, retrieved from https://www.gnpd.com/sinatra/recordpage/5988067/ Database accession no. 5988067**
- **DATABASE GNPD [online] MINTEL; 12 April 2019 (2019-04-12), ANONYMOUS: "CC All Over Face Primer", XP055951392, retrieved from https://www.gnpd.com/sinatra/recordpage/6466531/ Database accession no. 6466531**
- **DATABASE GNPD [online] MINTEL; 25 September 2013 (2013-09-25), ANONYMOUS: "Gel Shampoo Rinse-In", XP055951395, retrieved from https://www.gnpd.com/sinatra/recordpage/2286489/ Database accession no. 2286489**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Gegenstand der vorliegenden Anmeldung ist ein wasserfreies Mittel zum Färben von keratinischem Material, insbesondere menschlichen Haaren, welches mindestens ein Alkylenglycol einer bestimmten Formel (a1), mindestens ein Pigment (a2), mindestens ein aminofunktionalisiertes Silikonpolymer (a3) und mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen (a4) enthält.

Ein weiterer Gegenstand dieser Anmeldung ist ein Verfahren zum Färben von keratinischem Material, insbesondere menschlichen Haaren, wobei das zuvor beschriebene Mittel auf das keratinische Material aufgetragen und gegebenenfalls nach einer Einwirkzeit von 30 Sekunden bis 45 Minuten wieder ausgespült wird.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbemitteln und Färbeverfahren. In der letzten Zeit besonders im Fokus stehen Färbesysteme, die auf Pigmenten basieren.

In der Datenbank Mintel wird ein wasserfreies Haarbehandlungs-Produkt mit dem Namen "Powder Foundation Long Keep SP Refill" beschrieben (Reocrd ID 5988067). In der INCI dieses Produktes werden PEG-8, verschiedene Pigmente wie beispielsweise synthetisches Fluorphlogopite, Titandioxid, Zinkoxid und Eisenoxide sowie ein Aminosilikon aufgelistet.

Ein weiteres Produkt aus der Datenbank Mintel ist das Produkt "CG All Over Face Primer" (Record ID 6466531). Die INCI dieses Produktes umfasst kein Wasser, Caprylylglycol, diverse Pigmente und ein Amodimethicon.

Das in Mintel beschriebene Produkt mit dem Namen "Gel Shampoo Rinse-In" (Record ID 2286489) enthält Butylenglycol, die Pigmente CI 61570 und CI 15985 und Amodimethicon.

WO 2020/260097 A1 beschreibt wasserfreie Zusammensetzungen enthaltend mindestens ein Aminosilikon, ein Alkoxysiloxan einer Formel (VIII) sowie eine farbgebende Verbindung, bei der es sich auch um ein Pigment handeln kann.

Es war die Aufgabe der vorliegenden Erfindung, ein Färbmittel bereitzustellen, das es ermöglicht, Pigmente in extrem dauerhafter Weise auf den Haaren zu fixieren. Bei Anwendung des Mittels in einem Färbeverfahren sollten besonders intensive Färbeergebnisse mit einer guten Waschechtheit, einer guten Reibechtheit, einem guten Egalisiervermögen und einem besonders gleichmäßigen Farbresultat erzielt werden.

Überraschenderweise hat sich nun herausgestellt, dass die vorgenannte Aufgabe hervorragend gelöst werden kann, wenn keratinisches Material, insbesondere menschliche Haare, mit einem wasserfreien Mittel gefärbt wird, welches mindestens Alkylenglycol der Formel (AG) (a1), mindestens ein Pigment (a2), mindestens ein aminofunktionalisiertes Silikonpolymer (a3) und mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen (a4) enthält.

Ein erster Gegenstand der vorliegenden Erfindung ist ein wasserfreies Mittel zum Färben von keratinischem Material, insbesondere menschlichen Haaren, enthaltend

(a1) mindestens Alkylenglycol der Formel (AG)

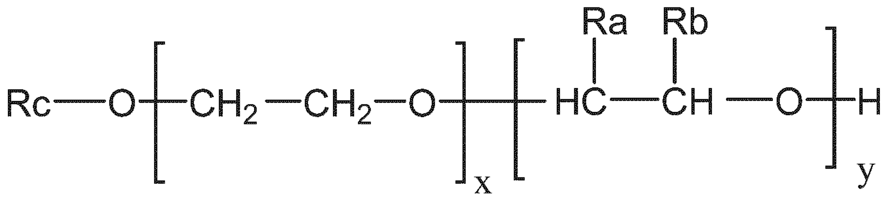

(AG),

wobei

- x für eine ganze Zahl von 0 bis 10000 steht,
- y für eine ganze Zahl von 0 bis 10000 steht,
- Ra, Rb unabhängig voneinander für ein Wasserstoffatom, eine $C_1$-$C_6$-Alkylgruppe oder eine Hydroxy-$C_1$-$C_6$-Alkylgruppe stehen,
- Rc für ein Wasserstoffatom, eine $C_1$-$C_6$-Alkylgruppe, eine Phenylgruppe oder eine Benzylgruppe steht,

mit der Maßgabe, dass x + y mindestens 1 ist, und
(a2) mindestens ein Pigment, und
(a3) mindestens ein aminofunktionalisiertes Silikonpolymer, und
(a4) mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen.

Im Rahmen der zu dieser Erfindung führenden Arbeiten konnte herausgefunden werden, dass ganz besonders gute Farbergebnisse erhalten werden konnten, wenn das oder die Pigmente in Abmischung mit mindestens einem Alkylenglycol der Formel (AG) und mindestens einem Aminosilikon in Form eines wasserfreien Systems auf das Kertinmaterial, insbesondere das menschliche Haar, appliziert wurden. Insbesondere die Waschechtheit und die Reibechtheit der gefärbten Haare konnten verbessert werden.

<u>keratinisches Material</u>

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.
Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

<u>Mittel zur Färbung</u>

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die Pigmente als farbgebende Verbindungen in einem besonders homogenen und gleichmäßigen Film an der Oberfläche des Keratinmaterials ab.
Das Färbemittel stellt erfindungsgemäß ein anwendungsbereites Mittel dar. Dieses anwendungsbereite Mittel kann beispielsweise in einen Container abgefüllt und in dieser Form ohne weitere Verdünnungs-, Mischungs- oder andere Verfahrens-Schritte auf das Keratinmaterial appliziert werden. Aus Gründen der Lagerstabilität hat sich jedoch als ganz besonders bevorzugt herausgestellt, wenn das anwendungsbereite kosmetische Mittel vom Friseur oder Anwender erst kurz vor der Anwendung hergestellt wird. Zur Herstellung des anwendungsbereiten Mittels kann beispielsweise das Gemisch oder die Vordispersion aus Alkylenglycol der Formel (AG) (a1) und Pigment (a2) mit einem oder mehreren weiteren Mitteln erfolgen, wobei eines dieser weiteren Mittel mindestens ein aminofunktionalisiertes Silikonpolymer (a3) enthält. Genauso ist jedoch auch denkbar, dass das anwendungsbereite Mittel durch Abmischung von mindestens drei verschiedenen Mitteln hergestellt wird, wobei eines dieser Mittel mindestens ein Alkylenglycol der Formel (AG) (a1), ein weiteres Mittel mindestens ein Pigment (a2) und noch ein weiteres Mittel mindestens aminofunktionalisiertes Silikonpolymer (a3) beinhaltet. Das Vermischen der Mittel kann zum Beispiel durch Verschütteln erfolgen und gewährleistet auf diese Weise eine ganz besonders gleichmäßige Verteilung der dispergierten Pigmente. Hierbei ist kennzeichnend für das anwendungsbereites Mittel, dass es wasserfrei ist.

wasserfreies Mittel

Das erfindungsgemäße Mittel ist wasserfrei. Unter dem Begriff wasserfrei wird im Sinne der vorliegenden Erfindung verstanden, dass dem Mittel intentionell kein Wasser als separater Bestandteil zugesetzt wird. Zur Erzielung bestimmter gewünschter anwendungstechnischer Eigenschaften kann es zwar von Vorteil sein, dem Mittel Inhaltsstoffe zuzusetzen, die hygroskopisch sind oder die Wasser als nicht vermeidbaren Nebenbestandteil enthalten. In diesem Fall wird unter wasserfrei verstanden, dass das Mittel - bezogen auf sein Gesamtgewicht - weniger als 1,0 Gew.-% Wasser, bevorzugt weniger als 0,5 Gew.-% Wasser, weiter bevorzugt weniger als 0,1 Gew.-% Wasser und ganz besonders bevorzugt weniger als 0,01 Gew.-% Wasser enthält. Explizit ganz besonders bevorzugt enthält das erfindungsgemäße Mittel 0 Gew.-% Wasser.

Anders ausgedrückt ist ein erster Gegenstand der vorliegenden Erfindung ist ein Mittel zum Färben von keratinischem Material, insbesondere menschlichen Haaren, enthaltend

(a1) mindestens Alkylenglycol der Formel (AG)

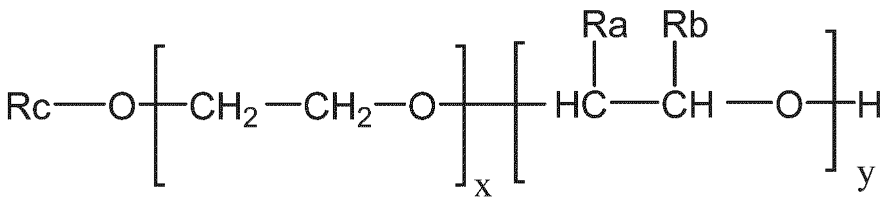

(AG),

wobei

x für eine ganze Zahl von 0 bis 10000 steht,
y für eine ganze Zahl von 0 bis 10000 steht,
Ra, Rb unabhängig voneinander für ein Wasserstoffatom, eine $C_1$-$C_6$-Alkylgruppe oder eine Hydroxy-$C_1$-$C_6$-Alkylgruppe stehen,
Rc für ein Wasserstoffatom, eine $C_1$-$C_6$-Alkylgruppe, eine Phenylgruppe oder eine Benzylgruppe steht,

mit der Maßgabe, dass x + y mindestens 1 ist, und
(a2) mindestens ein Pigment, und
(a3) mindestens ein aminofunktionalisiertes Silikonpolymer, und
(a4) mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen,

wobei das Mittel - bezogen auf sein Gesamtgewicht - weniger als 1,0 Gew.-% Wasser, bevorzugt weniger als 0,5 Gew.-% Wasser, weiter bevorzugt weniger als 0,1 Gew.-% Wasser, noch weiter bevorzugt weniger als 0,01 Gew.-% Wasser und ganz besonders bevorzugt 0 Gew.-% Wasser enthält.

Alkylenglycole der Formel (AG-I) (a1)

Als ersten erfindungswesentlichen Bestandteil (a1) enthält das erfindungsgemäße Mittel mindestens ein Alkylenglycol der Formel (AG)

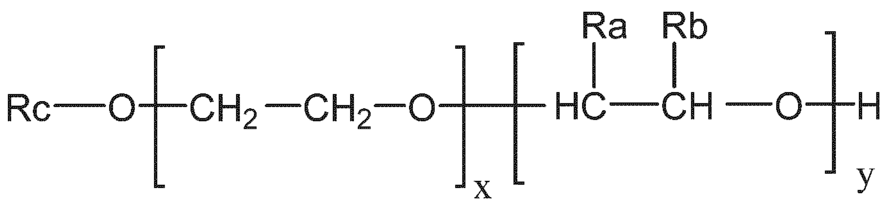

(AG),

wobei

x für eine ganze Zahl von 0 bis 10000 steht,
y für eine ganze Zahl von 0 bis 10000 steht,

Ra, Rb unabhängig voneinander für ein Wasserstoffatom, eine $C_1$-$C_6$-Alkylgruppe oder eine Hydroxy-$C_1$-$C_6$-Alkylgruppe stehen,

Rc für ein Wasserstoffatom, eine $C_1$-$C_6$-Alkylgruppe, eine Phenylgruppe oder eine Benzylgruppe steht,

mit der Maßgabe, dass x + y mindestens 1 ist, und

Überraschenderweise hat sich herausgestellt, dass der Einsatz mindestens eines speziellen Alkylenglycols der Formel (AG) die Waschbeständigkeit der Färbungen nach Anwendung des erfindungsgemäßen Mittel auf dem Keratinmaterial stark verbessert.

Bei den Alkylenglycolen der Formel (AG) handelt es sich um protische Substanzen mit mindestens einer Hydroxy-Gruppe. Die Substituenten Ra, Rb, Rc in den Verbindungen der Formel (AG-I) sind nachstehend beispielhaft erläutert:

Beispiele für eine $C_1$-$C_6$-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Bevorzugte Beispiele für eine Hydroxy-$C_1$-$C_6$-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; die Hydroxymethylgruppe und die 2-Hydroxyethylgruppe sind besonders bevorzugt.

Durch die Variation der Reste Ra, Rb und Rc sowie x und y können die Polarität des Alkylenglycols (AG) eingestellt und die Viskosität des erfindungsgemäßen Mittels beeinflusst werden.

In den Alkylenglycolen (a1) der Formel (AG) kann x für eine ganze Zahl von 0 bis 10000 stehen. Der Rest y kann für eine ganze Zahl von 0 bis 10000 stehen. Hierbei besteht die Maßgabe, dass die Summe aus x und y für einen Wert von mindestens 1 stehen muss. Wenn y für die Zahl 0 steht, sind die Reste Ra und Rb in den Alkylenglycolen der Formel (AG) nicht existent.

Der Rest $R_c$ steht für ein Wasserstoffatom, eine $C_1$-$C_6$-Alkylgruppe, eine Phenylgruppe oder eine Benzylgruppe. Im Rahmen dieser Ausführungsform ist es weiterhin ganz besonders bevorzugt, wenn Rc für ein Wasserstoffatom steht.

Der Einsatz bestimmter Alkylenglycole der Formel (AG) hat sich zur Erzeugung von Färbungen mit guter Waschechtheit und guter Reibechtheit als ganz besonders gut geeignet erwiesen. So ist im Rahmen dieser Ausführungsform der Einsatz von Polyethylenglycolen ganz besonders bevorzugt.

Polyethylenglycole sind Alkylenglycole der Formel (AG) bei welchen x für eine ganze Zahl von 1 bis 10000, insbesondere für eine ganze Zahl von 2 bis 10000, steht und y für die Zahl 0 steht. Rc steht für ein Wasserstoffatom.

Im Rahmen einer weiteren ganz besonders bevorzugten Ausführungsform ist ein erfindungsgemäßes Mittel dadurch gekennzeichnet, dass es

(a1) mindestens Alkylenglycol der Formel (AG) enthält, wobei

x für eine ganze Zahl von 1 bis 10000, steht, und

y für die Zahl 0 steht, und

Rc für ein Wasserstoffatom steht.

Bei den entsprechenden Polyethylenglycolen handelt es sich um die Verbindungen der Formel (AG-1),

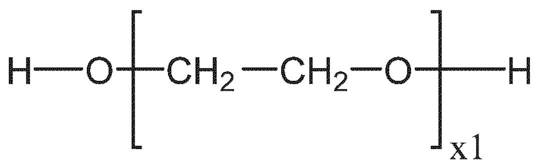

(AG-1),

wobei x1 für eine ganze Zahl von 1 bis 10000 steht.

Im Rahmen der zu dieser Erfindung führenden Arbeiten hat sich herausgestellt, dass die Polyethylenglycole eine besonders gute Eignung zeigen, um zum einen die Echtheitseigenschaften der Färbemittel zu verbessern und zum anderen um die Viskosität der Mittel optimal einzustellen.

Abhängig von ihrer Kettenlänge sind Polyethylenglycole flüssige oder feste, wasserlösliche Polymere. Polyethylenglycole mit einer Molekülmasse zwischen 200 g/mol und 400 g/mol sind bei Raumtemperatur nichtflüchtige Flüssigkeiten. PEG 600 weist einen Schmelzbereich von 17 bis 22 °C und somit eine pastenartige Konsistenz auf. Bei Molekülmassen über 3000 g/mol sind die PEG feste Substanzen und werden als Schuppen oder Pulver in den Handel gebracht.

Vor allem der Einsatz von niedermolekularen Polyethylenglycolen hat sich zur Lösung der erfindungsgemäßen Aufgabenstellung gut geeignet erwiesen. Bei niedermolekularen Polyethylenglycolen im Sinne der vorliegenden Erfindung steht x1 für eine ganze Zahl von 1 bis 100, bevorzugt für eine ganze Zahl von 1 bis 80, weiter bevorzugt für eine

ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15.

Im Rahmen einer weiteren ganz besonders bevorzugten Ausführungsform ist ein erfindungsgemäßes Mittel dadurch gekennzeichnet, dass es

(a1) mindestens ein Alkylenglycol der Formel (AG-1) enthält, wobei

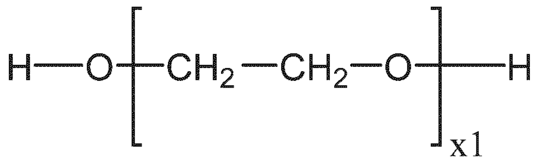

(AG-1),

x1 für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht.

Ein ganz besonders bevorzugtes niedermolekulares Polyethylenglycol ist beispielsweise PEG-8. PEG-8 umfasst 8 Ethylenglycol-Einheiten (x1 = 8), besitzt ein mittleres Molgewicht von 400 g/mol und trägt die CAS-Nummer 25322-68-3. PEG-8 wird alternativ auch als PEG 400 bezeichnet und ist beispielsweise von der Firma APS kommerziell erhältlich.

Weitere gut geeignete niedermolekulare Polyethylenglycole sind beispielsweise PEG-6, PEG-7, PEG-9 und PEG-10.

Ein weiteres gut geeignetes Polyethylenglycol ist beispielsweise PEG-32. PEG-32 umfasst 32 Ethylenglycol-Einheiten (x1 = 32), besitzt ein mittleres Molgewicht von 1500 g/mol und trägt die CAS-Nummer 25322-68-3. PEG-32 wird alternativ auch als PEG 1500 bezeichnet und kann zum Beispiel von der Firma Clariant kommerziell erworben werden.

Weiterhin hat sich auch der Einsatz von hochmolekularen Polyethylenglycolen zur Lösung der erfindungsgemäßen Aufgabenstellung gut geeignet erwiesen.

Hochmolekulare Polyethylenglycole im Sinne der vorliegenden Erfindung können durch die Formel (AG-2) dargestellt werden, wobei die Indexzahl x2 für eine ganze Zahl von 101 bis 10000 steht

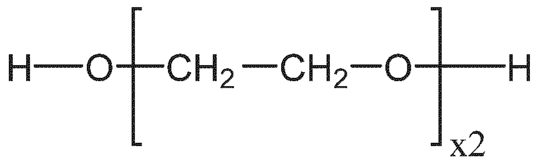

(AG-2).

Bei ganz besonders gut geeigneten hochmolekularen Polyethylenglycolen steht x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200.

Im Rahmen einer weiteren ganz besonders bevorzugten Ausführungsform ist ein erfindungsgemäßes Mittel dadurch gekennzeichnet, dass es

(a1) mindestens ein Alkylenglycol der Formel (AG-2) enthält, wobei

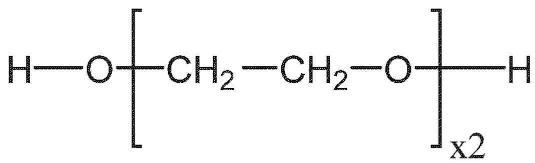

(AG-2),

x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

Ein besonders gut geeignetes hochmolekulares Polyethylenglycol ist beispielsweise PEG 6000, welches von der Firma National Starch (China) kommerziell erhalten werden kann. Das Molgewicht von PEG 6000 liegt bei 6000 bis

7500 g/mol, dies entspricht einem x2-Wert von 136 bis 171.

Ein weiteres gut geeignetes Polyethylenglycol ist PEG 12000, das zum Beispiel unter dem Handelsnamen Polyethylene Glycol 12000 S (oder PEG 12000 S) von der Firme CG Chemikalien kommerziell vertrieben wird. Das Molgewicht von PEG 12000 wird mit 10500 bis 15000 g/mol angegeben, entsprechend einem x2-Wert von 238 bis 341.

Ein weiteres gut geeignetes Polyethylenglycol ist auch PEG 20000, welches unter dem Handelsnamen Polyglycol 20000 P bzw. unter dem Alternativnamen PEG-350 von der Firma Clariant käuflich zu erwerblich ist. Für PEG 20000 wird ein Molgewicht von im Schnitt 20000 g/mol angegeben, dies entspricht einem x2-Wert von 454.

Überraschenderweise hat sich herausgestellt, dass Färbemittel, welche sowohl ein niedermolekulares Alkylenglycol (AG-1) als auch ein hochmolekulares Alkylenglycol (AG-2) enthalten, besonders gute anwendungstechnische Eigenschaften besitzen, da diese Mittel sowohl sehr gute Echtheitseigenschaften aufweisen als auch ein im Hinblick auf ihre rheologisches Profil optimiert sind.

Im Rahmen einer weiteren explizit ganz besonders bevorzugten Ausführungsform ist ein erfindungsgemäßes Mittel daher dadurch gekennzeichnet, dass es

(a11) mindestens ein erstes Alkylenglycol der Formel (AG-1) enthält, wobei

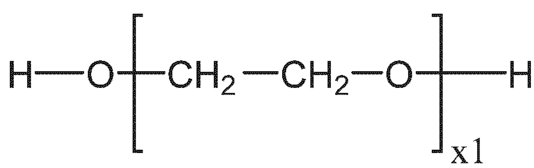

(AG-1),

x1 für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht, und

(a12) mindestens ein zweites Alkylenglycol der Formel (AG-2) enthält, wobei

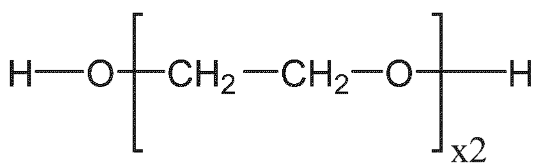

(AG-2),

x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

Im Rahmen einer weiteren Ausführungsform kann das erfindungsgemäße Mittel auch mindestens ein Alkylenglycol der Formel (AG-3) enthalten,

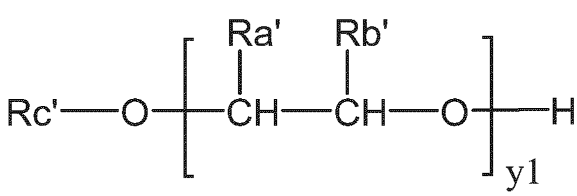

(AG-3),

wobei

y1 für eine ganze Zahl von 1 bis 1000, bevorzugt für eine ganze Zahl von 3 bis 100, besonders bevorzugt für eine ganze Zahl von 5 bis 50, steht,

Ra', Rb' unabhängig voneinander für ein Wasserstoffatom oder für eine $C_1$-$C_6$-Alkylgruppe stehen, wobei bevorzugt einer der Reste aus Ra' und Rb' für ein Wasserstoffatom steht und der verbleibende der beiden Reste für eine $C_1$-$C_6$-Alkylgruppe, bevorzugt für eine Methylgruppe, steht, und

Rc' für eine $C_1$-$C_6$-Alkylgruppe steht.

Im Rahmen einer weiteren bevorzugten Ausführungsform ist ein erfindungsgemäßes Mittel daher dadurch gekennzeichnet, dass es
(a1) mindestens Alkylenglycol der Formel (AG-3) enthält,

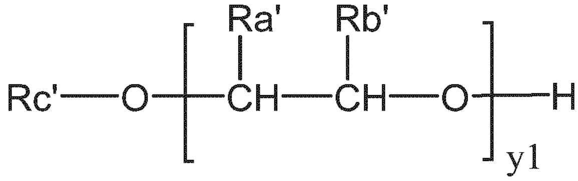

(AG-3),

wobei

- y1 für eine ganze Zahl von 1 bis 1000, bevorzugt für eine ganze Zahl von 3 bis 100, besonders bevorzugt für eine ganze Zahl von 5 bis 50, steht,
- Ra’, Rb’ unabhängig voneinander für ein Wasserstoffatom oder für eine $C_1$-$C_6$-Alkylgruppe stehen, wobei bevorzugt einer der Reste aus Ra’ und Rb’ für ein Wasserstoffatom steht und der verbleibende der beiden Reste für eine $C_1$-$C_6$-Alkylgruppe, bevorzugt für eine Methylgruppe, steht, und
- Rc’ für eine $C_1$-$C_6$-Alkylgruppe steht.

Bei einem ganz besonders gut geeigneten Alkylenglycol der Formel (AG-3) handelt es sich um Propylene Glycol Monobutylether, das auch als PPG-14 Butyl ether bezeichnet wird und die CAS-Nummer 9003-13-8. Trägt. PPG-14 Butyl ether kann kommerziell unter dem Handelsnamen Ucon Fluid AP von der Firma Dow erworben werden.

Im Rahmen einer weiteren ganz besonders bevorzugten Aufführungsform ist ein erfindungsgemäßes Mittel daher weiterhin dadurch gekennzeichnet dass es
(a1) PPG-14 Butyl ether enthält.

Das Alkylenglycol der Formel (AG-3) kann entweder als alleiniges Alkylenglycol oder auch in Kombination mit weiteren Alkylenglyclen, insbesondere den Alkylenglycolen der Formel (AG-1) und/oder (AG-2), eingesetzt werden.

Sehr gute Effekte lassen sich daher mit einem erfindungsgemäßen Mittel erhalten, welches dadurch gekennzeichnet ist, dass es

(a11) mindestens ein Alkylenglycol der Formel (AG-1) enthält, wobei

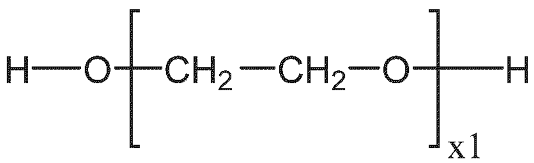

(AG-1),

- x1 für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht, und

(a13) mindestens Alkylenglycol der Formel (AG-3) enthält,

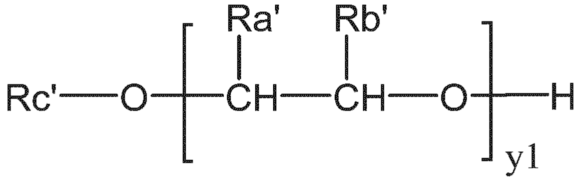

(AG-3),

wobei

- y1 für eine ganze Zahl von 1 bis 1000, bevorzugt für eine ganze Zahl von 3 bis 100, besonders bevorzugt für eine ganze Zahl von 5 bis 50, steht,

Ra', Rb' unabhängig voneinander für ein Wasserstoffatom oder für eine $C_1$-$C_6$-Alkylgruppe stehen, wobei bevorzugt einer der Reste aus Ra' und Rb' für ein Wasserstoffatom steht und der verbleibende der beiden Reste für eine $C_1$-$C_6$-Alkylgruppe, bevorzugt für eine Methylgruppe, steht, und

Rc' für eine $C_1$-$C_6$-Alkylgruppe steht.

Gute Effekte lassen sich auch mit einem erfindungsgemäßen Mittel erhalten,welches dadurch gekenneichnet ist, dass es

(a12) mindestens ein Alkylenglycol der Formel (AG-2) enthält, wobei

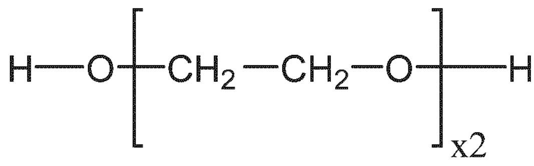

(AG-2),

x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht, und

(a13) mindestens Alkylenglycol der Formel (AG-3) enthält,

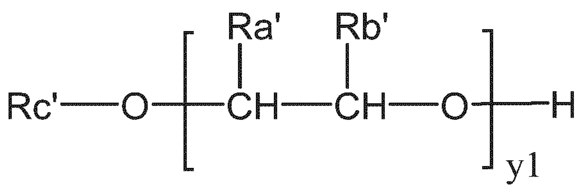

(AG-3),

wobei

y1 für eine ganze Zahl von 1 bis 1000, bevorzugt für eine ganze Zahl von 3 bis 100, besonders bevorzugt für eine ganze Zahl von 5 bis 50, steht,

Ra', Rb' unabhängig voneinander für ein Wasserstoffatom oder für eine $C_1$-$C_6$-Alkylgruppe stehen, wobei bevorzugt einer der Reste aus Ra' und Rb' für ein Wasserstoffatom steht und der verbleibende der beiden Reste für eine $C_1$-$C_6$-Alkylgruppe, bevorzugt für eine Methylgruppe, steht, und

Rc' für eine $C_1$-$C_6$-Alkylgruppe steht.

Sehr gute Effekte lassen sich auch mit einem erfindungsgemäßen Mittel erhalten,welches dadurch gekenneichnet ist, dass es

(a11) mindestens ein Alkylenglycol der Formel (AG-1) enthält, wobei

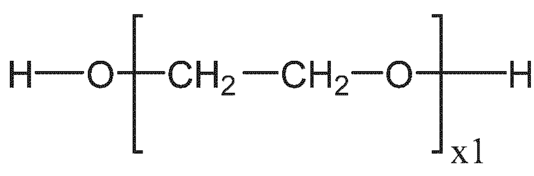

(AG-1),

x1 für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht, und

(a12) mindestens ein Alkylenglycol der Formel (AG-2) enthält, wobei

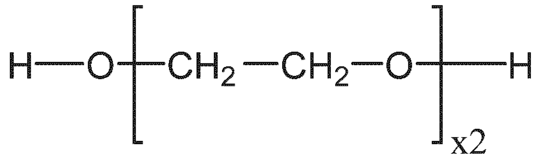

(AG-2),

x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht, und

(a13) mindestens Alkylenglycol der Formel (AG-3) enthält,

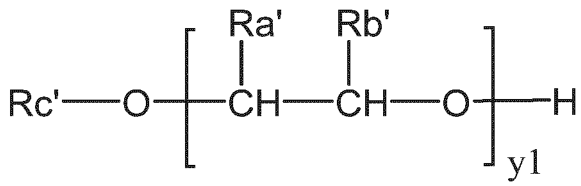

(AG-3),

wobei

y1 für eine ganze Zahl von 1 bis 1000, bevorzugt für eine ganze Zahl von 3 bis 100, besonders bevorzugt für eine ganze Zahl von 5 bis 50, steht,

Ra', Rb' unabhängig voneinander für ein Wasserstoffatom oder für eine $C_1$-$C_6$-Alkylgruppe stehen, wobei bevorzugt einer der Reste aus Ra' und Rb' für ein Wasserstoffatom steht und der verbleibende der beiden Reste für eine $C_1$-$C_6$-Alkylgruppe, bevorzugt für eine Methylgruppe, steht, und

Rc' für eine $C_1$-$C_6$-Alkylgruppe steht.

Da das erfindungsgemäße Mittel wasserfrei ist, müssen auch für den kosmetischen Träger des Mittels von Wasser verschiedene Bestandteile gewählt werden. In diesem Zusammenhang hat es sich als ganz besonders bevorzugt herausgestellt, wenn das oder die Alkylenglycole (AG), und hierbei insbesondere die besonders bevorzugten Verbindungen der Formel (AG-1), (AG-2) und/oder (AG-3), selbst als kosmetische Trägersubstanzen fungieren. Zu diesem Zweck werden die Alkylenglycole (AG) besonders bevorzugt in den entsprechend hohen Mengenbereichen im erfindungsgemäßen Mittel eingesetzt. Diese Einsatzmengen können beispielsweise - bezogen auf das Gesamtgewicht des Mittels - im Bereich von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% liegen.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels - ein oder mehrere Alkylenglycol der Formel (AG) (a1) in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

Das erfindungsgemäße Mittel enthält bevorzugt - bezogen auf das Gesamtgewicht des Mittels - (a11) ein oder mehrere Alkylenglycole der Formel (AG-1) in einer Gesamtmenge von 20,0 bis 99,0 Gew.-%, bevorzugt von 40,0 bis 95,0 Gew.-%, besonders bevorzugt von 60,0 bis 90,0 Gew.-%.

Das erfindungsgemäße Mittel enthält bevorzugt - bezogen auf das Gesamtgewicht des Mittels - (a12) es ein oder mehrere Alkylenglycole der Formel (AG-2) in einer Gesamtmenge von 1,0 bis 35,0 Gew.-%, bevorzugt von 3,0 bis 30,0 Gew.-%, besonders bevorzugt von 4,0 bis 25,0 Gew.-%.

Das erfindungsgemäße Mittel enthält bevorzugt - bezogen auf das Gesamtgewicht des Mittels - (a13) ein oder mehrere Alkylenglycole der Formel (AG-3) in einer Gesamtmenge von 0,1 bis 20,0 Gew-%, bevorzugt von 0,2 bis 10,0 Gew.-%, noch weiter bevorzugt von 0,4 bis 5,0 Gew.-%, noch weiter bevorzugt von 0,5 bis 3,0 Gew.-% und ganz besonders bevorzugt von 0,5 bis 1,5 Gew.-%.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels - (a1) ein oder mehrere Alkylenglycole der Formel (AG-1) in einer Gesamtmenge von 20,0 bis 99,0 Gew.-%, bevorzugt von 40,0 bis 95,0 Gew.-%, besonders bevorzugt von 60,0 bis 90,0 Gew.-%, enthält und/oder es ein oder mehrere Alkylenglycole der Formel (AG-2) in einer Gesamtmenge von 1,0 bis 35,0 Gew.-%, bevorzugt von 3,0 bis 30,0 Gew.-%, besonders bevorzugt von 4,0 bis 25,0 Gew.-%, enthält und/oder es ein oder mehrere Alkylenglycole der Formel (AG-3) in einer Gesamtmenge von 0,1 bis 20,0 Gew-%, bevorzugt von 0,2 bis 10,0 Gew.-%, besonders bevorzugt von 0,4 bis 5,0 Gew.-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 20,0 bis 99,0 Gew.-% enthält, und
(a12) ein oder mehrere Alkylenglycole der Formel (AG-2) in einer Gesamtmenge von 1,0 bis 35,0 Gew.-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 40,0 bis 95,0 Gew.-% enthält, und
(a12) ein oder mehrere Alkylenglycole der Formel (AG-2) in einer Gesamtmenge von 3,0 bis 30,0 Gew.-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 60,0 bis 90,0 Gew.-% enthält, und
(a12) ein oder mehrere Alkylenglycole der Formel (AG-2) in einer Gesamtmenge von 4,0 bis 25,0 Gew.-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 20,0 bis 99,0 Gew.-% enthält, und
(a12) ein oder mehrere Alkylenglycole der Formel (AG-2) in einer Gesamtmenge von 1,0 bis 35,0 Gew.-% enthält, und
(a13) ein oder mehrere Alkylenglycole der Formel (Ag-3) in einer Gesamtmenge von 0,1 bis 20,0 Gew-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 40,0 bis 95,0 Gew.-%enthält, und
(a12) ein oder mehrere Alkylenglycole der Formel (AG-2) in einer Gesamtmenge von 3,0 bis 30,0 Gew.-% enthält,
(a13) ein oder mehrere Alkylenglycole der Formel (Ag-3) in einer Gesamtmenge von 0,2 bis 10,0 Gew.-% enthält.

Im Rahmen einer weiteren ganz besonders bevorzugten Ausführungsform ist ein erfindungsgemäßes Mittel dadurch gekennzeichnet, dass es - bezogen auf das Gesamtgewicht des Mittels -

(a11) ein oder mehrere Alkylenglycole der Formel (AG-1) in einer Gesamtmenge von 60,0 bis 90,0 Gew.-% enthält, und
(a12) ein oder mehrere Alkylenglycole der Formel (AG-2) in einer Gesamtmenge von 4,0 bis 25,0 Gew.-% enthält, und
(a13) ein oder mehrere Alkylenglycole der Formel (Ag-3) in einer Gesamtmenge von 0,4 bis 5,0 Gew.-% enthält.

Hierbei versteht es sich, dass die Summe aus (a1) allen im Mitteln enthaltenen Alkylenglycolen (AG), Pigmenten (a2) und aminofunktionalisierten Silikonpolymeren (a3) bei nicht mehr als 100 Gew.-% liegen kann. Sind noch andere, optionale Inhaltsstoffe im Mittel enthalten, so verringert sich die Gesamtsumme aus (a1), (a2) und (a3) in entsprechendem Ausmaß auf Werte von unter 100 Gew.-%.

Pigmente (a2)

Als zweiten wesentlichen Bestandteil enthält das erfindungsgemäße Mittel mindestens ein Pigment (a2). Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C

erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens ein anorganisches Pigment (a2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)

Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch ein oder mehrere farbgebende Verbindungen (a2) aus der Gruppe der organischen Pigmente enthalten
Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Mittel dadurch gekennzeichnet, dass es mindestens ein organisches Pigment (a2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich

beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem erfindungsgemäßen Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 µm, vorzugsweise von 5,0 bis 45 µm, bevorzugt von 10 bis 40 µm, insbesondere von 14 bis 30 µm, aufweist. Die mittlere Teilchengröße $D_{50}$ kann beispielsweise unter Verwendung von dynamischer Lichtstreuung (DLS) bestimmt werden.

Die Pigmente (a2) stellen den zweiten wesentlichen des erfindungsgemäßen Mittels dar und werden bevorzugt in bestimmten Mengenbereichen im Mittel eingesetzt.

Besonders gute Ergebnisse wurden erhalten, wenn das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere anorganische Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere organische Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

<u>aminofunktionalisierte Silikonpolymere (a3)</u>

Als dritten erfindungswesentlichen Inhaltsstoff enthält das erfindungsgemäße Mittel mindestens ein aminofunktionalisiertes Silikonpolymer (a3). Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte aminofunktionalisierten Silikonpolymeren (a3) erzielt werden, wenn diese mindestens eine primäre, mindestens eine sekundäre und/oder mindestens eine tertiäre Aminogruppe tragen. Färbungen mit den höchsten Farbintensitäten wurden jedoch beobachtet, wenn ein aminofunktionalisiertes Silikonpolymer (a3) im Mittel eingesetzt wurde, welches mindestens eine sekundäre Aminogruppe enthält.

In einer ganz besonders bevorzugten Ausführungsform ist ein erfindungsgemäßes Mittel dadurch gekennzeichnet, dass es zusätzlich

(a3) mindestens ein aminofunktionalisiertes Silikonpolymer mit mindestens einer sekundären Aminogruppe, enthält.

Die sekundäre Aminogruppe(n) kann bzw. können sich an verschiedenen Positionen des aminofunktionalisierten Silikonpolymers befinden. Ganz besonders gute Effekt wurden gefunden, wenn ein aminofunktionalisiertes Silikonpolymer (a3) eingesetzt wurde, dass mindestens eine, bevorzugt mehrere Struktureinheiten der Formel (Si-Amino) besitzt.

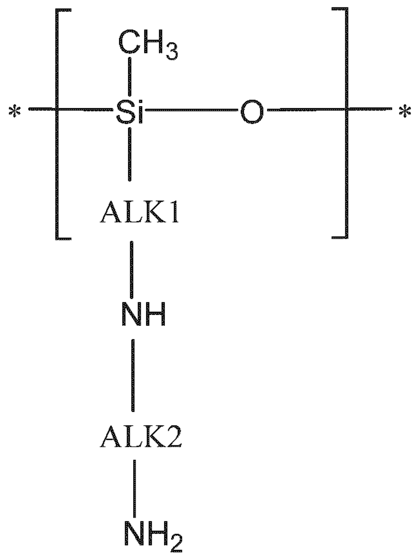

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Mittel dadurch gekennzeichnet, dass das Mittel mindestens ein aminofunktionalisiertes Silikonpolymer (a3) enthält, das mindestens eine Struktureinheit der Formel (Si-Amino) umfasst,

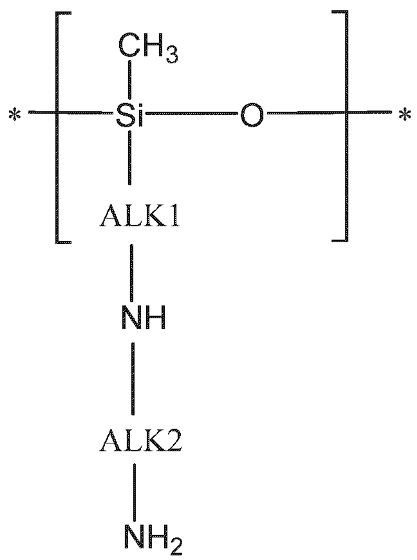

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alky-

lengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer (a3) dar, so dass das Silikonpolymer mehrer Struktureinheiten der Formel (Si-Amino) umfasst.

Im Folgenden werden besonders gut geeignete aminofunktionalisierte Silikonpolymere (a3) mit mindestens einer sekundären Aminogruppe aufgelistet.

Färbungen mit den allerhöchsten Farbintensitäten konnten erhalten werden, wenn ein Mittel auf dem keratinischen Material appliziert wurde, das mindestens ein aminofunktionalisiertes Silikonpolymer (a3) enthält, das Struktureinheiten der Formel (Si-I) und der Formel (Si-II) umfasst

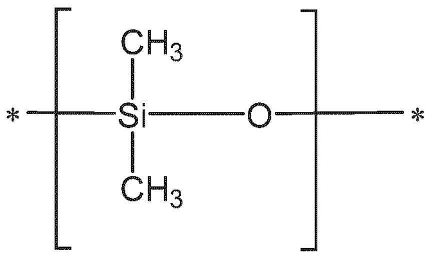

(Si-I)

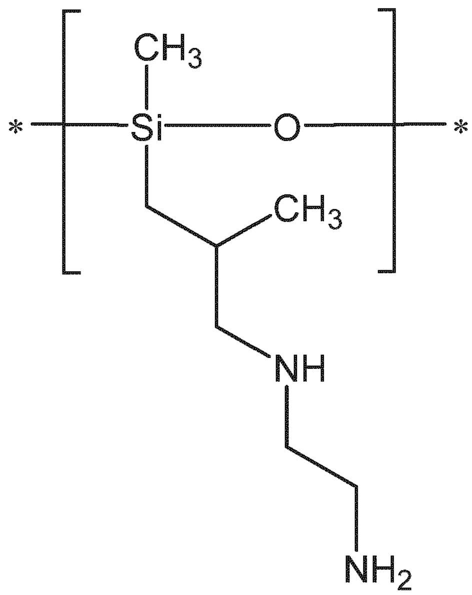

(Si-II).

In einer weiteren explizit ganz besonders bevorzugten Ausführungsform ist ein erfindungsgemäßes Mittel dadurch gekennzeichnet, dass es mindestens ein aminofunktionalisiertes Silikonpolymer (a3) enthält, das Struktureinheiten der Formel (Si-I) und der Formel (Si-II) umfasst

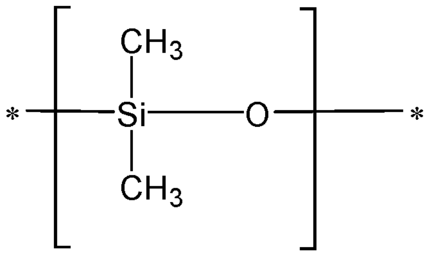

(Si-I)

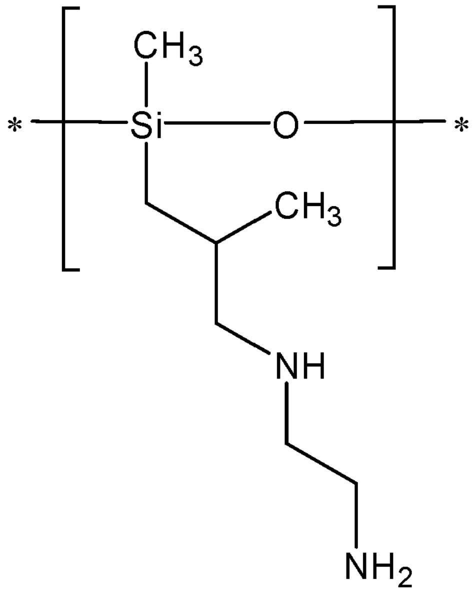

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres aminofunkionalisiertes Silikonpolymer mit den Struktureinheiten (Si-I) und (Si-II) ist beispielweise das Handelsprodukt DOWSIL™ AP-8568 Amino Fluid, das ebenfalls das von der Firma Dow Chemical Company komerziell vertrieben wird.

Im Rahmen einer weiteren bevorzugten Ausführungsform ist ein erfindungsgemäßes Mittel dadurch gekennzeichnet dass es mindestens ein aminofunktionelles Silikonpolymer (a3) der Formel der Formel (Si-III) enthält,

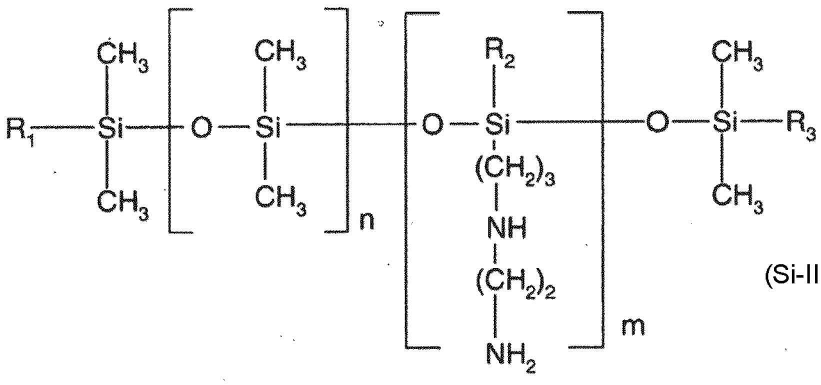

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Mittel sind gekennzeichnet durch ihren Gehalt an mindestens einem aminofunktionellen Silikonpolymer (a3) der Formel der Formel (Si-IV) enthält,

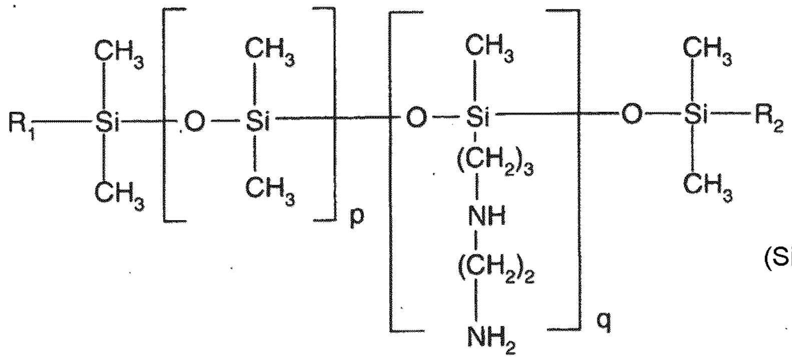

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_2$]-Gruppierung gebunden.

Als besonders wirkungsvoll im Hinblick auf die gewünschten Effekte haben sich auch erfindungsgemäße Mittel erwiesen, welche mindestens ein aminofunktionelles Silikonpolymer (a3) der Formel der Formel (Si-V) enthalten

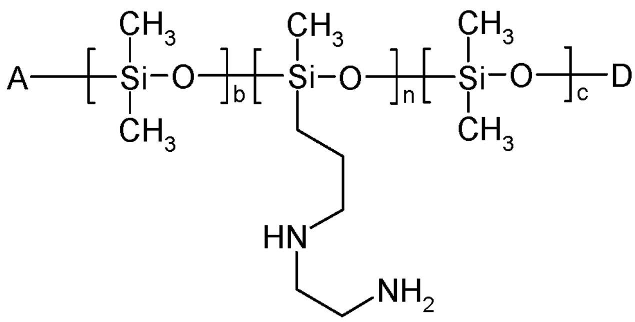

(Si-V),

in der

| | |
|---|---|
| A | für eine Gruppe -OH, -O-$Si(CH_3)_3$,-O-$Si(CH_3)_2OH$ ,-O-$Si(CH_3)_2OCH_3$ steht, |
| D | für eine Gruppe -H, -$Si(CH_3)_3$,-$Si(CH_3)_2OH$, -$Si(CH_3)_2OCH_3$ steht, |
| b, n und c | für ganze Zahlen zwischen 0 und 1000 stehen, |

mit den Maßgaben

- n > 0 und b + c > 0
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Mittel (a) kann weiterhin auch ein oder mehrere verschiedene aminofunktionalisierte Silikonpolymere enthalten, die durch die Formel (Si-VI)

$$M(R_aQ_bSiO_{(4-a-b)/2)x}(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\ CH_2CH_2-$, $-CH_2CH(CH_3)C(O)OCH_2-$, $-(CH_2)_3\ CC(O)OCH_2CH_2-$, $-C_6H\ _4C_6H_4-$, $-C_6H\ _4CH_2C_6H_4-$; und $-(CH\ _2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2\ )_zNH_2$, worin z 1 oder mehr ist. Eine andere mögliche Formel für Z ist $-NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist $-N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH\ _2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b\ SiO_{(4-a-b)/2}$-Einheiten zu den $R_cSiO\ _{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer weiteren bevorzugten Ausführungsform ist ein erfindungsgemäßes Mittel dadurch gekennzeichnet dass es mindestens ein aminofunktionelles Silikonpolymer der Formel (Si-VII) enthält,

$$R'_aG_{3-a}\text{-}Si(OSiG_2)n\text{-}(OSiG_bR'_{2-b})_m\text{-}O\text{-}SiG_{3-a}\text{-}R'_a \qquad \text{(Si-VII)},$$

worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$, $-\ CH_2CH_2CH_2CH_3$, $-O-CH_2CH(CH_3)_2$, $-CH_2CH(CH_3)_2$, $-O-CH(CH_3)CH_2CH_3$, $-\ CH(CH_3)CH_2CH_3$, $-O-C(CH_3)_3$, $-C(CH_3)_3$ ;
- a steht für eine Zahl zwischen 0 und 3, insbesondere 0;
- **b steht** für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus

    ◦ $-Q-N(R'')-CH_2-CH_2-N(R'')_2$
    ◦ $-Q-N(R'')_2$
    ◦ $-Q-N^+(R'')_3A^-$
    ◦ $-Q-N^+H(R'')_2\ A^-$
    ◦ $-Q-N^+H_2(R'')A^-$
    ◦ $-Q-N(R'')-CH_2-CH_2-N^+R''H_2A^-$,

    wobei jedes Q für eine chemische Bindung, $-CH_2-$, $-CH_2-CH_2-$, $-CH_2CH_2CH_2-$, $-C(CH_3)_2-$, $-CH_2CH_2CH_2CH_2-$, $-CH_2C(CH_3)_2-$, $-CH(CH_3)CH_2CH_2-$ steht,

R" für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Mittel dadurch gekennzeichnet, dass es mindestens ein aminofunktionelles Silikonpolymer (a3) der Formel (Si-VIIa) enthält,

$$(CH_3)_3Si\text{-}[O\text{-}Si(CH_3)_2]_n[OSi(CH_3)]_m\text{-}OSi(CH_3)_3 \qquad (Si\text{-}VIIa),$$

|

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Mittel dadurch gekennzeichnet, dass es mindestens ein aminofunktionelles Silikonpolymer der Formel (Si-VIIb) enthält

$$R\text{-}[Si(CH_3)_2\text{-}O]_{n1}[Si(R)\text{-}O]_m\text{-}[Si(CH_3)_2]_{n2}\text{-}R \qquad (Si\text{-}VIIb),$$

|

$$(CH_2)_3NH(CH_2)_2NH_2$$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Mittel bevorzugt, die ein aminofunktionelles Silikonpolymer (a3) enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Mittel geeignet, welche ein spezielles 4-Morpholinomethyl-substituiertes Silikonpolymer (a3) enthielten. Dieses aminofunktionalisierte Silikonpolymer umfasst Struktureinheiten der Formeln (SI-VIII) und der Formel (Si-IX)

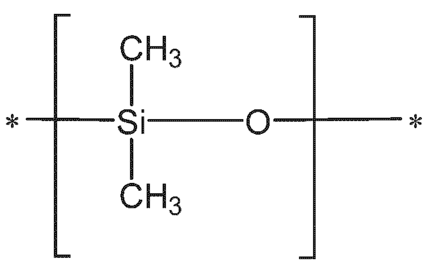

(Si-VIII)

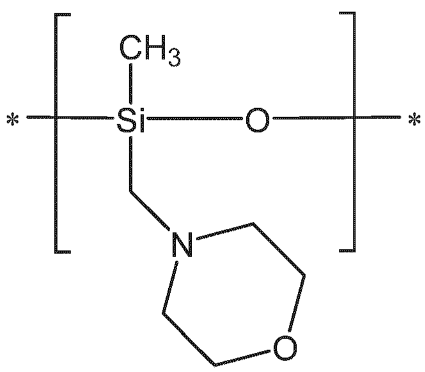

(Si-IX).

Entsprechende 4-Morpholinomethyl-substituiertes Silikonpolymere werden im folgenden beschrieben.

Ein bevorzugtes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell

erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist

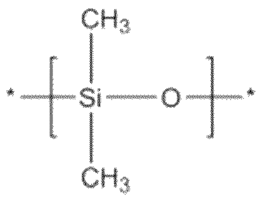
(Si-VIII),
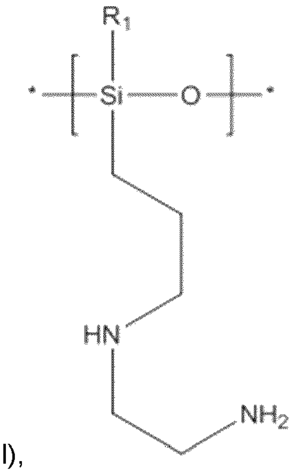
(Si-X)
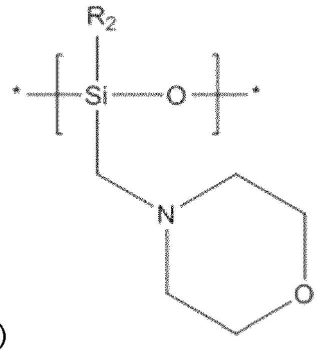
(Si-IX)

in denen

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.

Besonders bevorzugte erfindungsgemäße Mittel enthalten mindestens ein 4-morpholinomethylsubstituierten Silikons der Formel (Si-XI)

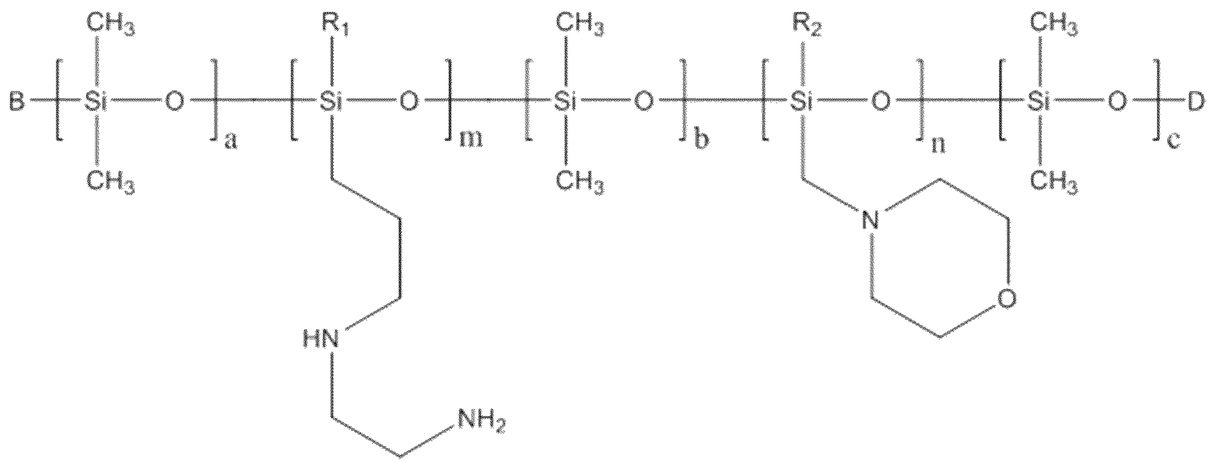

(Si-XI)

in der

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.
B für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,

a, b und c unabhängig voneinander für ganze Zahlen zwischen 0 und 1000 stehen, mit der Maßgabe $a + b + c > 0$
m und n unabhängig voneinander für ganze, Zahlen zwischen 1 und 1000 stehen mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | |
|---|---|
| B = $-O-Si(CH_3)_2OH$ und | D = $-Si(CH_3)_3$ |
| B = $-O-Si(CH_3)_2OH$ und | D = $-Si(CH_3)_2OH$ |
| B = $-O-Si(CH_3)_2OH$ und | D = $-Si(CH_3)_2OCH_3$ |
| B = $-O-Si(CH_3)_3$ | und D = $-Si(CH_3)_2OH$ |
| B = $-O-Si(CH_3)_2OCH_3$ | und D = $-Si(CH_3)_2OH$ |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Es hat sich als besonders vorteilhaft herausgestellt, wenn das erfindungsgemäße Mittel das oder die aminofunktionalisierten Silikonpolymere (a3) in bestimmten Mengenbereichen enhält. Besonders gute Ergebnisse konnten erhalten werden, wenn das Mittel - bezogen auf das Gesamtgewicht des Mittels - in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere aminofunktionalisierte Silikonpolymere (a3) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

## Anlagerungsprodukte von $C_1$-$C_6$-Alkylenoxid(en) an die Ester aus $C_{12}$-$C_{30}$-Fettsäuren und aromatischen $C_1$-$C_{12}$-Alkoholen (a4)

Zur weiteren Verbesserung der Echtheitseigenschaften wie Reibechtheit und Waschechtheit enthalten die erfindungsgemäßen Mittel als vierten erfindungswesentlichen Bestandteil zusätzlich mindestens ein Anlagerungsprodukt von $C_1$-$C_6$-Alkylenoxid(en) an die Ester aus $C_{12}$-$C_{30}$-Fettsäuren und aromatischen $C_1$-$C_{12}$-Alkoholen

Die Anlagerungsprodukte von $C_1$-$C_6$-Alkylenoxid(en) an die Ester aus $C_{12}$-$C_{30}$-Fettsäuren und aromatischen $C_1$-$C_{12}$-Alkoholen (a4) werden im Folgenden verkürzt auch als alkoxylierte Fettsäureester (a4) bezeichnet.

Bei $C_1$-$C_6$-Alkylenoxiden handelt es sich um die Epoxide von $C_1$-$C_6$-Alkanen. Als besonders gut geeignete $C_1$-$C_6$-Alkylenoxide können beispielsweise Ethylenoxid (1,2-Epoxyethan), Propylenoxid (1,2-Epoxypropan) und Butylenoxide (1,2-Epoxybutan sowie 2,3-Epoxybutan) genannt werden.

Die ethoxylierten Fettsäure-Ester (a4) basieren auf $C_{12}$-$C_{30}$-Fettsäuren. Bei diesen erfindungsgemäßen $C_{12}$-$C_{30}$-Fettsäuren handelt es sich um lineare oder verzweigte, gesättigte oder einfach oder mehrfach ungesättigte Fettsäuren, die auch eine oder mehrere Hydroxygruppen tragen können. Die erfindungsgemäßen $C_{12}$-$C_{24}$-Fettsäuren sind dadurch gekennzeichnet, dass die 12 bis 30 Kohlenstoffatome, bevorzugt 12 bis 24 Kohlenstoffatome, umfassen. Weiterhin tragen die $C_{12}$-$C_{24}$-Fettsäuren mindestens eine Carbonsäure-Gruppierung.

Zur Ausbildung der erfindungsgemäßen ethoxylierten Fettsäureester (a4) können beispielsweise eine oder mehrere Fettsäuren eingesetzt werden, die ausgewählt sind aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z, 12Z, 15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure], Nervonsäure [(15Z)-Tetracos-15-ensäure] und/oder Rizinusölsäure ((9Z,12R)-12-Hydroxy-9-octadecensaeure.

Die ethoxylierten Fettsäure-Ester (a4) stellen Anlagerungsprodukte von $C_1$-$C_6$-Alkylenoxid(en) an die Ester der zuvor beschriebenen $C_{12}$-$C_{24}$-Fettsäuren und aromatischen $C_1$-$C_{12}$-Alkoholen dar. Kennzeichnend für die aromatischen $C_1$-$C_{12}$-Alkohole ist, dass sie 1 bis 12 Kohlenstoffatome und mindestens ein aromatisches Ringsystem umfassen. Die aromatische $C_1$-$C_{12}$-Alkohole besitzen jeweils mindestens eine Hydroxy-Gruppe, die sich entweder direkt am Aromaten

befinden kann (wie z.B. bei Phenol) oder aber über eine aliphatische Einheit mit dem Aromaten verknüpft sein kann (wie z.B. bei Benzylalkohol oder 2-Phenoxyethanol). Die Strukturen der aromatischen $C_1$-$C_{12}$-Alkohole können auch noch weitere Heteroatome wie Sauerstoff oder Stickstoff umfassen.

Die entsprechenden aromatischen $C_1$-$C_{12}$-Alkohole können ein- oder mehrwertige Alkohole sein, d.h. die Alkohole können eine oder mehrere Hydroxygruppen besitzen.

Einwertige aromatische $C_1$-$C_{12}$-Alkohole sind ganz besonders bevorzugt. Diese Verbindungsklasse umfasst genau eine Hydroxygruppe. Als geeignete Vertreter können beispielsweise Phenol, Benzylalkohol, 2-Phenylethylalkohol und 2-Phenoxyethanol genannt werden.

Besonders gute Waschechtheiten und Reibechtheiten konnten erhalten werden, wenn im erfindungsgemäßen Mittel mindestens ein Anlagerungsprodukt (a4) von $C_1$-$C_6$-Alkylenoxid(en) an einen Ester eingesetzt wurde, der durch Veresterung von einer $C_{12}$-$C_{30}$-Fettsäure mit einem aromatischen $C_1$-$C_{12}$-Alkohol aus der Gruppe aus Benzylalkohol, Phenol, 2-Phenylethylalkohol und 2-Phenoxyethanol, erhalten wird.

Im Rahmen einer weiteren bevorzugten Ausführungsform ist ein erfindungsgemäßes Mittel dadurch gekennzeichnet, dass es

(a4) mindestens ein Anlagerungsprodukt von $C_1$-$C_6$-Alkylenoxid(en) an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol, ganz besonders bevorzugt Benzylalkohol, erhalten wird.

Im Rahmen einer besonders bevorzugten Ausführungsform ist ein erfindungsgemäßes Mittel dadurch gekennzeichnet, dass es

(a4) mindestens ein Anlagerungsprodukt von $C_1$-$C_6$-Alkylenoxid(en) an einen Ester enthält, der durch Veresterung von einer $C_{12}$-$C_{24}$-Fettsäure mit Benzylalkohol erhalten wird.

Im Rahmen einer weiteren bevorzugten Ausführungsform ist ein erfindungsgemäßes Mittel dadurch gekennzeichnet, dass es

(a4) mindestens ein Anlagerungsprodukt von Ethylenoxid und/oder Propylenoxid an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol erhalten wird.

Im Rahmen einer weiteren besonders bevorzugten Ausführungsform ist ein erfindungsgemäßes Mittel dadurch gekennzeichnet, dass es

(a4) mindestens ein Anlagerungsprodukt von Ethylenoxid und/oder Propylenoxid an einen Ester enthält, der durch Veresterung von einer $C_{12}$-$C_{24}$-Fettsäure mit Benzylalkohol erhalten wird.

Wie bereits zuvor beschrieben können als besonders gut geeignete $C_1$-$C_6$-Alkylenoxide beispielsweise Ethylenoxid (1,2-Epoxyethan), das Propylenoxid (1,2-Epoxypropan) und Butylenoxide (1,2-Epoxybutan sowie 2,3-Epoxybutan) eingesetzt werden. Ethylenoxid (1,2-Epoxyethan) und Propylenoxid (1,2-Epoxypropan) sind explizit ganz besonders bevorzugt. Am allermeisten bevorzugt ist Propylenoxid (1,2-Epoxypropan).

Ein entsprechendes Anlagerungsprodukt von Ethylenoxid an die Ester aus $C_{12}$-$C_{30}$-Fettsäuren und aromatischen $C_1$-$C_{12}$-Alkoholen (a4) entsteht, wenn die $C_{12}$-$C_{30}$-Fettsäure selbst oder derbereits aus ihr ausgebildete Ester mit Ethylenoxid (Alternativname 1,2-Epoxyethan, CAS-Nummer 75-21-8) umgesetzt wird.

Analog entsteht ein entsprechendes Anlagerungsprodukt von Propylenoxid an die Ester aus $C_{12}$-$C_{30}$-Fettsäuren und aromatischen $C_1$-$C_{12}$-Alkoholen (a3), wenn die $C_{12}$-$C_{30}$-Fettsäure selbst oder der bereits aus ihr ausgebildete Ester mit Propylenoxid (Alternativname 1,2-Epoxypropan, CAS-Nummern 75-56-9 (Racemat), 15448-47-2 ((R)-Enantiomer, 16088-62-3 (S)-Enantiomer) umgesetzt wird.

Wird die $C_{12}$-$C_{30}$-Fettsäure selbst mit Ethylenoxid umgesetzt, so kann sich zunächst ein Addukt ausgehend von der Carbonsäure-Gruppierung der $C_{12}$-$C_{30}$-Fettsäure und dem Ethylenoxid ausbilden, so dass eine Gruppierung $*\text{-C(O)-O-CH}_2\text{-CH}_2\text{-O-}*$ entsteht. Bei dieser Gruppierung handelt es sich ebenfalls um einen Ester. Wird pro Mol Fettsäure ein Mol Ethylenoxid umgesetzt, so bildet sich im Mittel ein einfaches Addukt mit einer Einheit *-CH2-CH2-O-* aus. Abhängig davon, in welchem molaren Überschuss das Ethylenoxid eingesetzt wird, können sich jedoch auch mehrfache Addukte ausbilden, wobei pro Mol $C_{12}$-$C_{30}$-Fettsäure mehrere Einheiten *-CH2-CH2-O-* vorliegen. Zur Ausbildung des Esters (a3) wird dieses Addukt dann weiterhin mit mindestens einem aromatischen $C_1$-$C_{12}$-Alkohol umgesetzt. Die mit einem Stern gekennzeichneten Positionen stellen hierbei die Bindung zum restlichen Teil der Fettsäure und die Bindung mit dem restlichen Teil des Alkohols dar.

Wird die $C_{12}$-$C_{30}$-Fettsäure analog mit Propylenoxid umgesetzt, so kann sich zunächst ein Addukt ausgehend von der Carbonsäure-Gruppierung der $C_{12}$-$C_{30}$-Fettsäure und dem Propylenoxid ausbilden, so dass eine Gruppierung $*\text{-C(O)-O-CH(CH}_3\text{)-CH}_2\text{-O-}*$ oder eine Gruppierung $*\text{-C(O)-O-CH}_2\text{-CH(CH}_3\text{)-O-}*$. Im Reaktionsgemisch wird üblicherweise eine Mischung der beiden vorgenannten Gruppierungen erhalten. Bei beiden Gruppierungen handelt es sich ebenfalls um Ester. Wird pro Mol Fettsäure ein Mol Propylenoxid umgesetzt, so bildet sich im Mittel ein einfaches Addukt mit einem Gemisch der Einheiten $*\text{-CH(CH}_3\text{)-CH}_2\text{-O-}*$ und $*\text{-CH}_2\text{-CH(CH}_3\text{)-O-}*$ aus. Abhängig davon, in welchem molaren Überschuss das Propylenoxid eingesetzt wird, können sich jedoch auch mehrfache Addukte ausbilden, wobei pro Mol $C_{12}$-$C_{30}$-Fettsäure dann mehrere Einheiten $*\text{-CH(CH}_3\text{)-CH}_2\text{-O-}*$ und/oder $*\text{-CH}_2\text{-CH(CH}_3\text{)-O-}*$ vorliegen. Zur

Ausbildung des Esters (a3) wird dieses Addukt dann weiterhin mit mindestens einem aromatischen $C_1$-$C_{12}$-Alkohol umgesetzt. Die mit einem Stern gekennzeichneten Positionen stellen hierbei die Bindung zum restlichen Teil der Fettsäure und die Bindung mit dem restlichen Teil des Alkohols dar.

Weiterhin ist es prinzipiell denkbar, dass die $C_{12}$-$C_{30}$-Fettsäure mit einer Mischung aus Ethylenoxid und Propylenoxid umgesetzt wird. In diesem Fall bilden sich Gemische der zuvor beschriebenen Addukte aus. Auch die Umsetzungen von höheren Alkylenoxiden wie beispielsweise Butylenoxiden sind auf diese Weise mit den $C_{12}$-$C_{30}$-Fettsäuren möglich.

Die auf diese Weise hergestellten Anlagerungsprodukte führen beispielsweise zu den alkoxylierten Fettsäureestern der allgemeinen Formel (AFE-I)

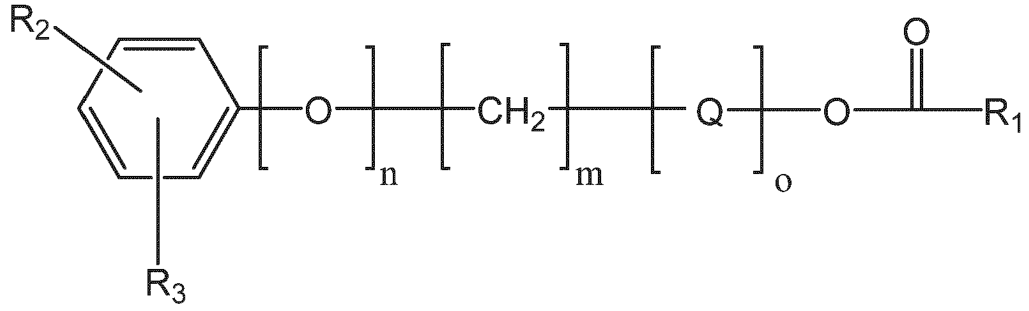

(AFE-I)

wobei

| | |
|---|---|
| R1 | für eine gesättigte oder ungesättigte $C_{11}$-$C_{29}$-Alkylgruppe steht |
| R2, R3 | unabhängig voneinander für ein Wasserstoffatom, eine $C_1$-$C_6$-Alkylgruppe, eine Hydroxygruppe oder eine $C_1$-$C_6$-Alkoxygruppe stehen, |
| n | für die Zahl 0 oder 1 steht, |
| m | für eine ganze Zahl von 0 bis 6 steht, |
| o | für eine ganze Zahl von 1 bis 60 steht, und |
| Q | für eine Struktureinheit $-O-CH_2-CH_2-$ , $-O-CH(CH_3)-CH_2-$ oder $-O-CH_2-CH(CH_3)-$ steht, |

wobei die Maßgabe besteht, dass, wenn m gleich 0 ist, auch n gleich 0 ist.

Mit Mitteln, die mindestens ethoxylierten Fettsäureester (a4) der Formel (AFE-I) enthielten, wurden Färbungen erhalten, die sich im Hinblick auf gute Waschechtheiten und gute Reibechtheiten besonders auszeichneten. Aus diesem Grund ist der Einsatz einer oder mehrerer ethoxylierter Fettsäureester (a4) der Formel (AFE-I) in den Mitteln als weiterer Bestandteil zusätzlich zu den Bestandteilen (a1), (a2) und (a3) ganz besonders bevorzugt.

Im Rahmen einer weiteren besonders bevorzugten Ausführungsform ist ein erfindungsgemäßes Mittel dadurch gekennzeichnet, dass es

(a4) mindestens einen alkoxylierten Fettsäureester der allgemeinen Formel (AFE-I) enthält

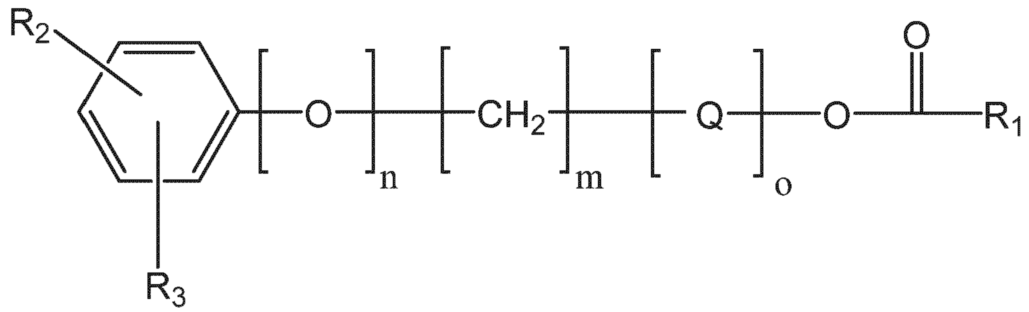

(AFE-I)

wobei

| | |
|---|---|
| R1 | für eine gesättigte oder ungesättigte $C_{11}$-$C_{29}$-Alkylgruppe steht |
| R2, R3 | unabhängig voneinander für ein Wasserstoffatom, eine $C_1$-$C_6$-Alkylgruppe, eine Hydroxygruppe oder eine |

$C_1$-$C_6$-Alkoxygruppe stehen,

n für die Zahl 0 oder 1 steht,

m Q für eine ganze Zahl von 0 bis 6 steht,

o für eine ganze Zahl von 1 bis 60 steht, und für eine Struktureinheit $-O-CH_2-CH_2-$ , $-O-CH(CH_3)-CH_2-$ oder $-O-CH_2-CH(CH_3)-$ steht,

wobei die Maßgabe besteht, dass, wenn m gleich 0 ist, auch n gleich 0 ist.

Der Rest R1 steht für eine gesättigte oder ungesättigte $C_{11}$-$C_{29}$-Alkylgruppe. Eine ungesättigte $C_{11}$-$C_{23}$-Alkylgruppe kann eine oder mehrere Doppelbindungen umfassen und wird alternativ auch als ungesättigte $C_{11}$-$C_{23}$-Alkenylgruppe bezeichnet. Die gesättigte oder ungesättigte $C_{11}$-$C_{29}$-Alkylgruppe kann linear oder verzweigt sein.

Bevorzugt steht R1 für eine lineare, gesättigte oder ungesättigte $C_{11}$-$C_{23}$-Alkylgruppe.

Die Reste R2 und R3 stehen voneinander für ein Wasserstoffatom, eine $C_1$-$C_6$-Alkylgruppe, eine Hydroxygruppe oder eine $C_1$-$C_6$-Alkoxygruppe. Ganz besonders bevorzugt stehen die Reste R2 und R3 beide für ein Wasserstoffatom.

Die Index-Zahl n steht für die Zahl 0 oder 1. Bevorzugt steht n für die Zahl 0.

Die Index-Zahl m steht für eine ganze Zahl von 0 bis 6. Bevorzugt steht m für die Zahl 1.

Die Index-Zahl o steht für eine ganze Zahl von 1 bis 60. Bevorzugt steht o für eine ganze Zahl von 1 bis 30, weiter bevorzugt von 1 bis 20, noch weiter bevorzugt von 1 bis 10 und ganz besonders bevorzugt von 1 bis 5.

Der Rest Q steht für eine Struktureinheit $-O-CH_2-CH_2-$ , $-O-CH(CH_3)-CH_2-$ oder $-O-CH_2-CH(CH_3)$-steht. Besonders bevorzugt steht Q für eine Struktureinheit $-O-CH(CH_3)-CH_2-$ oder $-O-CH_2-CH(CH_3)-$.

Wenn o für eine Zahl von größer als 1 steht, sind in den Verbindungen der Formel (AFE-I) (bzw. auch der Formel (AFE-II) mehrere Struktureinheiten Q enthalten, in diesem Fall kann jede Struktureinheit Q von den anderen Struktureinheiten Q unabhängig gewählt werden.

Demzufolge ist ein bevorzugtes erfindungsgemäßes Mittel dadurch gekennzeichnet, dass es

(a4) mindestens einen alkoxylierten Fettsäureester der allgemeinen Formel (AFE-I) enthält

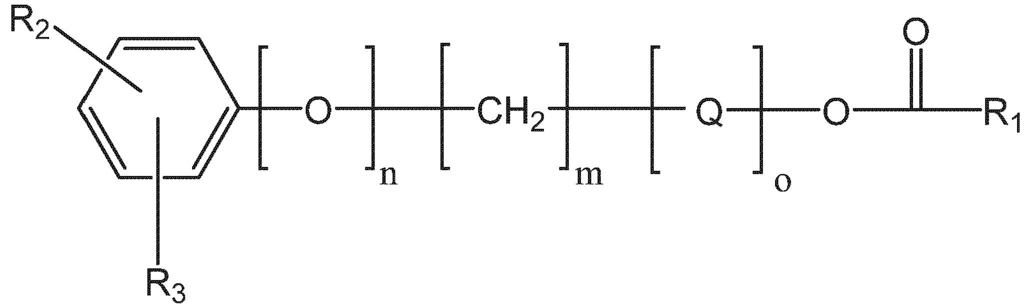

(AFE-I)

wobei

R1 für eine gesättigte oder ungesättigte $C_{11}$-$C_{29}$-Alkylgruppe steht

R2, R3 unabhängig voneinander für ein Wasserstoffatom, eine $C_1$-$C_6$-Alkylgruppe, eine Hydroxygruppe oder eine $C_1$-$C_6$-Alkoxygruppe stehen,

n für die Zahl 0 oder 1 steht,

m für eine ganze Zahl von 0 bis 6 steht,

o für eine ganze Zahl von 1 bis 60 steht, und

Q für eine Struktureinheit $-O-CH_2-CH_2-$ , $-O-CH(CH_3)-CH_2-$ oder $-O-CH_2-CH(CH_3)-$ steht,

wobei die Maßgabe besteht, dass, wenn m gleich 0 ist, auch n gleich 0 ist,
und wobei im Fall von o größer 1 jede Struktureinheit Q unabhängig von den anderen Struktureinheiten Q gewählt werden kann.

Zusammenfassend wurden mit den erfindungsgemäßen Mitteln besonders gute Ergebnisse erhalten, welche (a4) mindestens einen alkoxylierten Fettsäureester der allgemeinen Formel (AFE-I) enthielten,

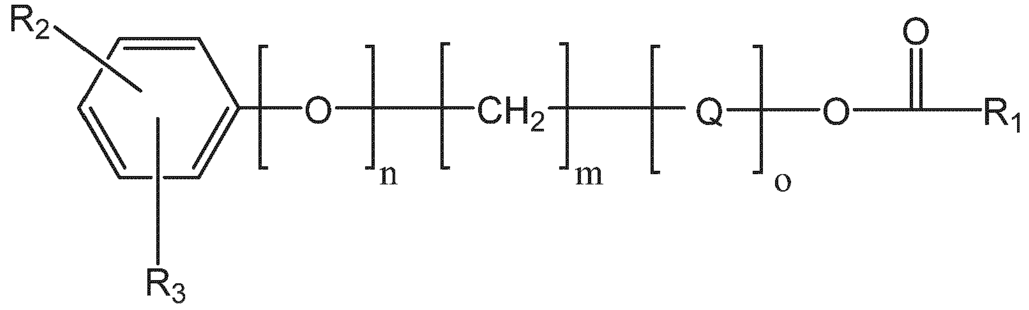

(AFE-I)

wobei

| | |
|---|---|
| R1 | für eine gesättigte oder ungesättigte $C_{11}$-$C_{29}$-Alkylgruppe steht |
| R2, R3 | beide für ein Wasserstoffatom stehen, |
| n | für die Zahl 0 steht, |
| m | für die Zahl 1 steht, |
| o | für eine ganze Zahl von 1 bis 30, bevorzugt von 1 bis 20, weiter bevorzugt von 1 bis 10 und ganz besonders bevorzugt von 1 bis 5 steht, und |
| Q | für eine Struktureinheit $-O-CH(CH_3)-CH_2-$ oder $-O-CH_2-CH(CH_3)-$ steht. |

Im Rahmen einer weiteren ganz besonders bevorzugten Ausführungsform ist ein erfindungsgemäßes Mittel dadurch gekennzeichnet, dass es
(a4) mindestens einen alkoxylierten Fettsäureester der allgemeinen Formel (AFE-I) enthält

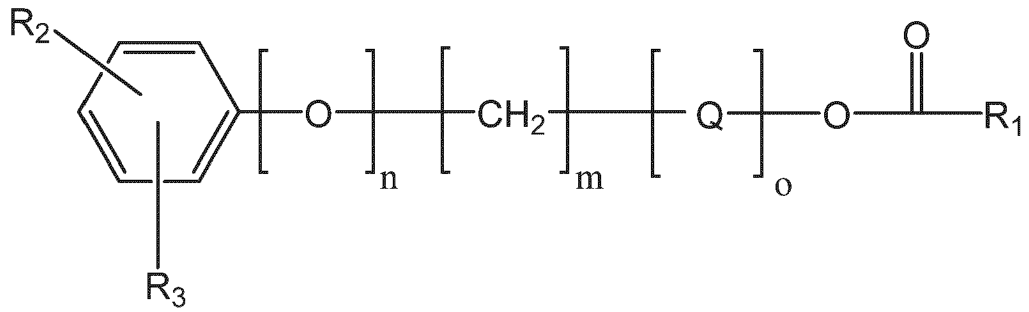

(AFE-I)

wobei

| | |
|---|---|
| R1 | für eine gesättigte oder ungesättigte $C_{11}$-$C_{29}$-Alkylgruppe steht |
| R2, R3 | beide für ein Wasserstoffatom stehen, |
| n | für die Zahl 0 steht, |
| m | für die Zahl 1 steht, |
| o | für eine ganze Zahl von 1 bis 30, bevorzugt von 1 bis 20, weiter bevorzugt von 1 bis 10 und ganz besonders bevorzugt von 1 bis 5 steht, und |
| Q | für eine Struktureinheit $-O-CH(CH_3)-CH_2-$ oder $-O-CH_2-CH(CH_3)-$ steht. |

Die ganz besonders bevorzugten alkoxylierten Fettsäureester dieser Ausführungsform fallen auch unter die allgemeine Formel (AFE-II)

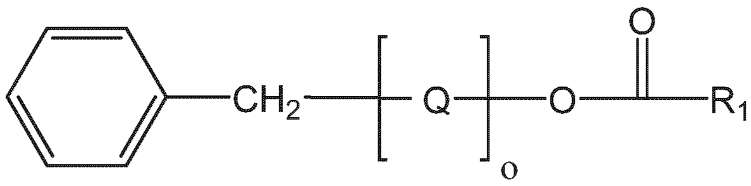

(AFE-II)

wobei

R1 für eine gesättigte oder ungesättigte $C_{11}$-$C_{29}$-Alkylgruppe steht
o für eine ganze Zahl von 1 bis 30, bevorzugt von 1 bis 20, weiter bevorzugt von 1 bis 10 und ganz besonders bevorzugt von 1 bis 5 steht, und
Q für eine Struktureinheit $-O-CH(CH_3)-CH_2-$ oder $-O-CH_2-CH(CH_3)-$ steht.

Im Rahmen einer weiteren ganz besonders bevorzugten Ausführungsform ist ein erfindungsgemäßes Mittel dadurch gekennzeichnet, dass es
(a4) mindestens einen alkoxylierten Fettsäureester der allgemeinen Formel (AFE-II) enthält

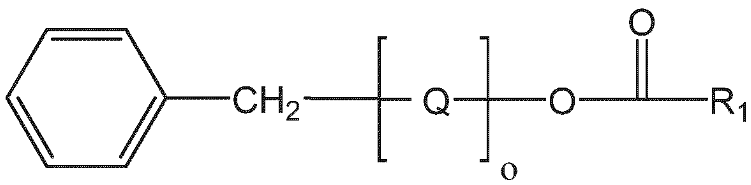

(AFE-II)

wobei

R1 für eine gesättigte oder ungesättigte $C_{11}$-$C_{29}$-Alkylgruppe steht
o für eine ganze Zahl von 1 bis 30, bevorzugt von 1 bis 20, weiter bevorzugt von 1 bis 10 und ganz besonders bevorzugt von 1 bis 5 steht, und
Q für eine Struktureinheit $-O-CH(CH_3)-CH_2-$ oder $-O-CH_2-CH(CH_3)-$ steht.

Auch hier kann im Fall von o größer 1 jede Struktureinheit Q unabhängig von den anderen Struktureinheiten Q gewählt werden kann.
Hierbei sind die Struktureinheiten Q sind in den alkoxylierten Fettsäureestern der allgemeinen Formel (AFE-II) so ausgerichtet, dass sich das Sauerstoffatom in der Gruppierung $-O-CH(CH_3)-CH_2-$ bzw. $-O-CH_2-CH(CH_3)-$ zur Benzylgruppe benachbart befindet, und die jeweilige Einheit - CH2- bzw. -CH(CH3)- an die Estergruppe -O-C(O)-R1 angrenzt.
Bei einer explizit ganz besonders gut geeigneten Verbindung dieses Typs handelt es sich um PPG-3 Benzyl Ether Myristate, das alternativ auch als α-(1-Oxotetradecyl)-ω-(phenylmethoxy) Poly[oxy(methyl-1,2-ethanediyl)] bezeichnet wird und die CAS-Nummer 642443-86-5 trägt.
PPG-3 Benzyl Ether Myristate kann zum Beispiel unter dem Handelsnamen Crodamol STS von der Firma Croda käuflich erworben werden.
Im Rahmen einer weiteren ganz besonders bevorzugten Ausführungsform ist ein erfindungsgemäßes Mittel dadurch gekennzeichnet, dass es
(a4) PPG-3 Benzyl Ether Myristate enthält.
Die alkoxylierten Fettsäureester (a4) werden besonders bevorzugt in bestimmten Mengenbereichen im erfindungsgemäßen Mittel eingesetzt.
Besonders gute Ergebnisse wurden erhalten, wenn das Mittel - bezogen auf das Gesamtgewicht des Mittels - ein oder mehrere alkoxylierte Fettsäureester (a4) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 8,0 Gew.-% und ganz besonders bevorzugt von 1,0 bis 5,0 Gew.-% enthält.
Im Rahmen einer weiteren bevorzugten Ausführungsform ist ein erfindungsgemäßes Mittel dadurch gekennzeichnet, dass es - bezogen auf das Gesamtgewicht des Mittels - ein oder mehrere alkoxylierte Fettsäureester (a4) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 8,0 Gew.-% und ganz besonders bevorzugt von 1,0 bis 5,0 Gew.-% enthält.

weitere optionale Bestandteile im Mittel

Abhängig von der gewünschten Form der Konfektionierung kann das erfindungsgemäße Mittel optional auch noch weitere Bestandteile oder Inhaltsstoffe enthalten.

Fettbestandteile im Mittel

Wenn das Mittel in Form einer Emulsion oder Creme zur Verfügung gestellt werden soll, hat sich der Einsatz mindestens eines Fettbestandteils als besonders vorteilhaft erwiesen. Bei den Fettbestandteilen handelt es sich um hydrophobe Substanzen, die in Gegenwart von Wasser unter Ausbildung von Mizell-Systemen Emulsionen formen können. Ohne auf diese Theorie festgelegt zu sein, wird vermutet, dass die $C_1$-$C_6$-Alkoxysilane - entweder in Form ihrer Monomere oder gegebenenfalls in Form ihrer kondensierten Oligomere - in diese hydrophobe Umgebung bzw. in die Mizell-Systeme eingebettet werden, so dass sich die Polarität ihrer Umgebung verändert. Bedingt durch den hydrophoben Charakter der Fettbestandteile wird auch die Umgebung der $C_1$-$C_6$-Alkoxysilane hydrophobiert. Es wird angenommen, dass die zum Film bzw. Coating führende Polymerisationsreaktion der $C_1$-$C_6$-Alkoxy-silane in einem Milieu verringerter Polarität mit reduzierter Geschwindigkeit abläuft.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt können die im Mittel zusätzlich eingesetzten Fettbestandteile ausgewählt werden aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe.

Im Rahmen einer weiteren bevorzugten Ausführungsform ist ein erfindungsgemäßes Mittel dadurch gekennzeichnet, dass es einen oder mehrere Fettbestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

Als ganz besonders bevorzugte Fettbestandteile werden in diesem Zusammenhang die Bestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{30}$-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte $C_{12}$-$C_{30}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9Z)-Octadec-9-en-1-ol (Oleylalkohol), (9E)-Octadec-9-en-1-ol (Elaidylalkohol), (9Z,12Z)-Octadeca-9,12-dien-1-ol (Linoleylalkohol), (9Z,12Z,15Z)-Octadeca-9,12,15-trien-1-ol (Linolenoylalkohol), Gadoleylalkohol ((9Z)-Eicos-9-en-1-ol), Arachidonalkohol ((5Z,8Z,11Z,14Z)-Eicosa-5,8,11,14-tetraen-1-ol), Erucylalkohol ((132)-Docos-13-en-1-ol) und/oder Brassidylalkohol ((13E)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Im Rahmen einer weiteren bevorzugten Ausführungsform ist ein erfindungsgemäßes Mittel dadurch gekennzeichnet, dass es einen oder mehrere $C_{12}$-$C_{30}$-Fettalkohole (a4) aus der Gruppe aus

Dodecan-1-ol (Dodecylalkohol, Laurylalkohol),
Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9Z)-Octadec-9-en-1-ol (Oleylalkohol),

(9*E*)-Octadec-9-en-1-ol (Elaidylalkohol),
(9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),
Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13E)-Docosen-1-ol),
2-Octyl-dodecanol,
2-Hexyl-Dodecanol und/oder
2-Butyl-dodecanol enthält.

Es hat sich als ganz besonders bevorzugt erwiesen, ein oder mehrere $C_{12}$-$C_{30}$-Fettalkohole in ganz bestimmten Mengenbereichen einzusetzen.

Des weiteren ist es ganz besonders bevorzugt, wenn die das Mittel - bezogen auf das Gesamtgewicht des Mittels- einen oder mehrere $C_{12}$-$C_{30}$-Fettalkohole in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin als ganz geeigneten Fettbestandteil kann das Mittel auch mindestens ein $C_{12}$-$C_{30}$-Fettsäuretriglycerid, der $C_{12}$-$C_{30}$-Fettsäuremonoglycerid und/oder $C_{12}$-$C_{30}$-Fettsäurediglycerid enthalten. Unter einem $C_{12}$-$C_{30}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{30}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{30}$-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die $C_{12}$-$C_{30}$-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)- Tetracos-15-ensäure].

Unter einem $C_{12}$-$C_{30}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Ganz besonders gute Ergebnisse wurden erhalten, wenn das Mittel mindestens ein $C_{12}$-$C_{30}$-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)-Tetracos-15-ensäure].

Im Rahmen einer weiteren Ausführungsform ist ein erfindungsgemäßes Mittel dadurch gekennzeichnet, dass es mindestens ein $C_{12}$-$C_{30}$-Fettsäuremonoglycerid enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Es hat sich als bevorzugt erwiesen, ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in ganz bestimmten Mengenbereichen im Mittel einzusetzen.

Im Hinblick auf die Lösung der erfindungsgemäßen Aufgabenstellung hat es sich als vorteilhaft erwiesen, wenn das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (a4) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die $C_{12}$-$C_{30}$-Fettsäuremono-, $C_{12}$-$C_{30}$-Fettsäuredi- und/oder $C_{12}$-$C_{30}$-Fettsäuretriglyceride können als alleinige Fettbestandteile (a4) in den Mitteln eingesetzt werden. Es kann sich jedoch auch erfindungsgemäß, mindestens ein $C_{12}$-$C_{30}$-Fettsäuremono-, $C_{12}$-$C_{30}$-Fettsäuredi- und/oder $C_{12}$-$C_{30}$-Fettsäuretriglycerid in Kombination mit mindestens einem $C_{12}$-$C_{30}$-Fettalkohol in das Mittel einzuarbeiten.

Weiterhin als ganz besonders bevorzugten Fettbestandteil kann das Mittel auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn das Mittel mindestens einen Kohlenwasserstoff enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Mittel dadurch gekennzeichnet, dass es mindestens einen Fettbestandteil aus der Gruppe der Kohlenwasserstoffe enthält.

Im Hinblick auf die Lösung der erfindungsgemäßen Aufgabenstellung hat es sich als ganz besonders bevozugt erwiesen, wenn das Mittel - bezogen auf das Gesamtgewicht des Mittels - einen oder mehrere Kohlenwasserstoffe in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthielt.

<u>weitere Inhaltsstoffe im Mittel</u>

Die Mittel können ferner auch noch weitere Wirk-, Hilfs- und Zusatzstoffe enthalten, wie beispielsweise Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; Polymere wie anionische, nichtionische und kationische Polymere; Tenside wie anionische, nichtionische, kationische, zwitterionische und amphotere Tenside, Fettbestandteile, faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

Verfahren zum Färben von Keratinmaterial

Die zuvor beschriebenen Mittel lassen sich hervorragend in Verfahren zum Färben von keratinischem Material, insbesondere von menschlichen Haaren, einsetzen.

Ein zweiter Gegenstand der vorliegenden Erfindung ist daher ein Verfahren zum Färben von keratinischem Material, insbesondere menschlichen Haaren, bei welchem ein Mittel, wie es bei der Beschreibung des ersten Erfindungsgegenstands im Detail offenbart wurde, auf die keratinischen Fasern aufgetragen wird und gegebenenfalls nach einer Einwirkzeit von 30 Sekunden bis 45 Minuten wieder ausgespült wird.

Mit anderen Worten ist ein zweiter Gegenstand der Erfindung ein Verfahren zum Färben von keratinischem Material, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,
(2) Einwirken des Färbemittels auf dem keratinischen Material und
(3) Ausspülen des Färbemittels mit Wasser.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.

In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das

(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

Im Anschluss an das Einwirken der Anwendungsmischung auf das Keratinmaterial wird diese schließlich in Schritt (3) des Verfahrens mit Wasser ausgespült.

Hierbei kann die Anwendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen des Mittels in Schritt (3) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das

(3) Ausspülen des Färbemittels ausschließlich mit Wasser.

<u>Verfahren zum Färben von Keratinmaterial, bei welchem zunächst das anwendungsbereite Mittel hergestellt wird.</u>

Wie bereits zuvor beschrieben, handelt es sich bei dem Mittel des ersten Erfindungsgegenstands um ein anwendungsbereites Mittel, das dem Anwender entweder direkt in seiner anwendungsbereiten Form zur Verfügung gestellt wird, oder aber das erst kurz vor der Anwendung durch Vermischen von verschiedenen Mitteln hergestellt wird.

Um eine besonders feine Verteilung der Pigmente zu gewährleisten, hat es sich als ganz besonders bevorzugt herausgestellt, das anwendungsbereite Mittel kurz vor der Anwendung durch Vermischen von zwei oder drei verschiedenen Mitteln herzustellen.

Im Rahmen einer besonders bevorzugten Ausführungsform wird das anwendungsbereite Mittel demnach durch Vermischen von mindestens zwei verschiedenen Mitteln hergestellt, wobei das erste dieser beiden Mittel das Gemisch aus mindestens Alkylenglycol der Formel (AG) (a1) und Pigment(en) (a2) umfasst. So kann das Gemisch aus Alkylenglycol der Formel (AG) (a1) und Pigment(en) (a2) beispielsweise eine Vordispersion darstellen. Das zweite Mittel enthält mindestens ein aminofunkionalisiertes Silikonpolymer (a3). Zur Herstellung des anwendungsbereiten Mittes werden die beiden vorgenannten Mittel dann miteinander verschüttelt oder verrührt.

Bevorzugt ist daher ein Verfahren zum Färben von keratinischem Material, insbesondere menschlichen Haaren, umfassend die folgenden Schritte:

(1) Bereitstellung eines wasserfreien Mittels (I), wobei das Mittel (I) enthält:

(a1) mindestens ein Alkylenglycol der Formel (AG) und
(a2) mindestens Pigment,

(2) Bereitstellung eines wasserfreien Mittels (II), wobei das Mittel (II) enthält:
(a3) mindestens ein aminofunktionalisiertes Silikonpolymer.
(3) Herstellung einer Anwendungsmischung durch Vermischen der Mittel (I) und (II),
(4) Applizieren der in Schritt (3) hergestellten Anwendungsmischung auf dem keratinischen Material,
(5) Einwirken der in Schritt (4) applizierten Anwendungsmischung auf dem keratinischen Material und
(6) Ausspülen der Anwendungsmischung mit Wasser,

wobei die Inhaltsstoffe (a1), (a2) und (a3) bereits im Detail offenbart wurden.

Das Mittel (I) stellt hierbei bevorzugt eine Vordispersion der Pigmente (a2) in dem oder den Alkylenglycol der Formel (AG) (a1) dar, die beispielsweise in Form eines Konzentrats vorliegen kann.

Werden die Mittel (I) und (II) vermischt, so gewährleistet das Vordispergieren der Pigmente (a2) im Alkylenglycol der Formel (AG) (a1) eine besonders feine Verteilung der Pigmente, die auch nach Vermischen der beiden Mittel (I) und (II) im anwendungsbereiten Mittel erhalten bleibt.

Schließlich kann es sich im Rahmen einer weiteren Ausführungsform als vorteilhaft erweisen, auch die Bestadteile (a1) und (a2) voneinander zu trennen, so dass mit der Herstellung des anwendungsbereiten Mittels die drei zuvor voneinander getrennten Bestandteile (a1), (a2) und (a3) miteinaner vermischt werden. Diese Herstellungform kann beispielsweise dann von Vorteil sein, wenn das oder die Pigmente (a2) in geringeren Mengen und/oder in Form eines Pulvers eingesetzt werden sollen. Die Konfektionierung der Pigmente in Pulverform vereinfacht die quantitative Überführung der Pigmente in ein Mischgefäß oder einen anderen Container.

Bevorzugt ist damit ein Verfahren zum Färben von keratinischem Material, insbesondere menschlichen Haaren, umfassend die folgenden Schritte:

(1) Bereitstellung eines wasserfreien Mittels (I), wobei das Mittel (I) enthält:
(a1) mindestens ein Alkylenglycol der Formel (AG),
(2) Bereitstellung eines wasserfreien Mittels (II), wobei das Mittel (II) enthält:
(a2) mindestens Pigment,
(3) Bereitstellung eines wasserfreien Mittels (III), wobei das Mittel (III) enthält:
(a3) mindestens ein aminofunktionalisiertes Silikonpolymer,
(4) Herstellung einer Anwendungsmischung durch Vermischen der Mittel (I) und (II) und (III),
(5) Applizieren der in Schritt (4) hergestellten Anwendungsmischung auf dem keratinischen Material,
(6) Einwirken der in Schritt (5) applizierten Anwendungsmischung auf dem keratinischen Material und
(7) Ausspülen der Anwendungsmischung mit Wasser,

wobei die Inhaltsstoffe (a1), (a2) und (a3) bereits im Detail offenbart wurden.

Hierbei enthält das Mittel (I) mindestens ein Alkylenglycol der Formel (AG) (a1) und kann beispielsweise in Form eines Konzentrats vorliegen.

Das Mittel (II) enthält mindestens ein Pigment (a2). In einer möglichen Form der Konfektionierung wird das Pigment beispielsweise in Form eines Pulvers bereitgestellt.

Das Mittel (III) enthält mindestens ein aminofunktionalisiertes Silikonpolymer (a3) und liegt bevorzugt in Form eines Konzentrats vor.

Betreffend die weiteren bevorzugten Ausführungsformen der erfindungsgemäßen Verfahren gilt *mutatis mutantis* das zum erfindungsgemäßen Mittel gesagte.

Beispiele

1. Formulierungen

Es wurden die folgenden anwendungsbereiten Färbemittel hergestellt (alle Angaben, sofern nichts anderes angegeben ist, in Gewichtsprozent):

| anwendungsbereites Färbemittel | E1 | E2 |
|---|---|---|
| PPG-3 Benzyl Ether Myristate (Crodamol STS) (a4) | 3,40 | 3,40 |
| PPG-14 Butyl ether (UCON FLUID AP, Dow Corning) (a13) | --- | 1,00 |
| Unipure Red LC 3071 (CI 15850, Sensient) (a2) | 0,38 | 0,38 |
| Aminosilikon (Dow Corning 2-8566 (Siloxanes and Silicones, 3-[(2-Aminoethyl)amino]-2-methylpropyl Me, Di-Me-Siloxane) (a3) | 0,74 | 0,74 |
| PEG 6000 (a12) (Polyethylengyclol, Molekulargewicht 6000 bis 7500 g/mol) | 9,93 | 9,93 |
| PEG 400 (a11) (Polyethylenglycol, Molekulargewicht 400 g/mol) | ad 100 | Ad 100 |

2. Anwendung

Die zuvor hergestellten anwendungsbereiten Mittel E1 und E2 wurden jeweils auf Haarsträhnen (Firma Kerling, Typ "Euronatur-haar weiß" (ENH)) aufgetragen (Flottenverhältnis: 1 g Mittel pro g Haarsträhne) und für drei Minuten einwirken gelassen. Im Anschluss daran wurden die Haarsträhnen gründlich (1 Minute) mit Wasser ausgewaschen und getrocknet.

3. Messung der Waschechtheit

Nach dem Trocknen wurden die gefärbten Strähnen mit einem Farbmessgerät der Firma Datacolor, Typ Spectraflash 450 vermessen.

Danach wurde jede Strähne dreimal mit der Hand gewaschen. Hierzu wurde jede Strähne mit Wasser angefeuchtet, danach wurde ein handelsübliches Shampoo (Schauma 7-Kräuter) auf die Strähne appliziert (0,25 g Shampoo pro 1 g Haar) und 30 Sekunden mit den Fingern einmassiert. Dann wurde die Strähne für 1 Minute unter fließendem, lauwarmem Wasser ausgespült und die Haarsträhne getrocknet. Der zuvor beschriebene Vorgang entspricht einer Haarwäsche. Für jede weitere Haarwäsche wurde der Vorgang wiederholt. Nach 3 Haarwäschen bzw. nach 6 Haarwäschen wurden die Strähnen erneut farbmetrisch vermessen.

Der für die Beurteilung der Waschechtheit herangezogene dE-Wert ergibt sich aus den an der jeweiligen Strähne gemessenen L*a*b*-Farbmesswerten wie folgt:

$$dE = [\,(L_i - L_0)^2 + (a_i - a_0)^2 + (b_i - b_0)]^{1/2}$$

$L_0$, $a_0$ und $b_0$ = Messwerte direkt nach der Färbung
$L_i$, $a_i$ und $b_i$ = Messwerte nach 3 Haarwäschen (bzw. nach 6 Haarwäschen)

Je kleiner der dE-Wert ist, desto geringer ist der Farbabstand im Vergleich zum gefärbten, nicht gewaschenen Haar und desto besser ist die Waschechtheit.

FA beschreibt den prozentualen Farberhalt nach der entsprechenden Anzahl an Haarwäschen und wird nach der folgenden Formel berechnet:

$$FA\,[\%] = 100 * \left(\frac{\Delta E\,(u-g)}{\Delta E(u-c)}\right)$$

FA = Farberhalt in [%]

u = unbehandeltes Ausgangshaar

g = gewaschenes coloriertes Haar

c = coloriertes Haar

Je höher der Farberhalt ist, desto besser ist die Waschechtheit.

| | E1 | E2 |
|---|---|---|
| dE (uncoloriert versus coloriert) | 60,40 | 61,10 |
| dE (coloriert, nach 3 Haarwäschen) | 1,20 | 3,40 |
| FA nach 3 Haarwäschen [%] | 100,00 | 105,00 |
| dE (coloriert, nach 6 Haarwäschen) | 1,20 | 6,30 |
| FA nach 6 Haarwäschen [%] | 102,00 | 98,00 |

Mit den Mitteln E1 und E2 konnten die Haarsträhnen intensiv gefärbt werden, besaßen eine sehr gute Waschechtheit und einen hohen Farberhalt.

## Patentansprüche

**1.** Wasserfreies Mittel zum Färben von keratinischem Material, insbesondere menschlichen Haaren, enthaltend

(a1) mindestens Alkylenglycol der Formel (AG)

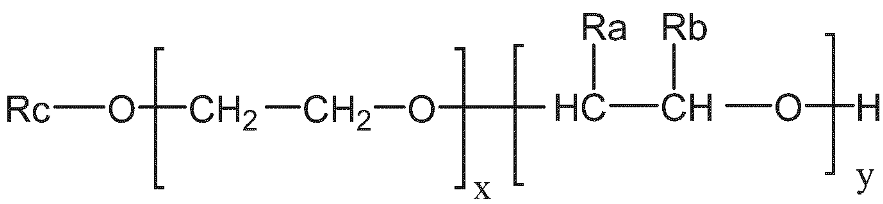

(AG),

wobei

x für eine ganze Zahl von 0 bis 10000 steht,
y für eine ganze Zahl von 0 bis 10000 steht,
Ra, Rb unabhängig voneinander für ein Wasserstoffatom, eine $C_1$-$C_6$-Alkylgruppe oder eine Hydroxy-$C_1$-$C_6$-Alkylgruppe stehen,
Rc für ein Wasserstoffatom, eine $C_1$-$C_6$-Alkylgruppe, eine Phenylgruppe oder eine Benzylgruppe steht,

mit der Maßgabe, dass x + y mindestens 1 ist, und
(a2) mindestens ein Pigment, und
(a3) mindestens ein aminofunktionalisiertes Silikonpolymer, und
(a4) mindestens ein Anlagerungsprodukt von $C_1$-$C_6$-Alkylenoxid(en) an die Ester aus $C_{12}$-$C_{30}$-Fettsäuren und

aromatischen $C_1$-$C_{12}$-Alkoholen.

**2.** Mittel nach Anspruch 1, **dadurch gekennzeichnet, dass** es
(a1) mindestens Alkylenglycol der Formel (AG) enthält, wobei

x für eine ganze Zahl von 1 bis 10000, steht, und
y für die Zahl 0 steht, und
Rc für ein Wasserstoffatom steht.

**3.** Mittel nach einem der Ansprüche 1 bis 2, **dadurch gekennzeichnet, dass** es
(a1) mindestens ein Alkylenglycol der Formel (AG-1) enthält, wobei

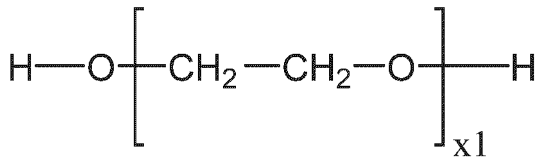

(AG-1),

x1 für eine ganze Zahl von 1 bis 100, bevorzugt für eine ganze Zahl von 1 bis 80, weiter bevorzugt für eine ganze Zahl von 2 bis 60, noch weiter bevorzugt für eine ganze Zahl von 3 bis 40, noch weiter bevorzugt für eine ganze Zahl von 4 bis 20 und ganz besonders bevorzugt für eine ganze Zahl von 6 bis 15 steht.

**4.** Mittel nach einem der Ansprüche 1 bis 3, **dadurch gekennzeichnet, dass** es
(a1) mindestens ein Alkylenglycol der Formel (AG-2) enthält, wobei

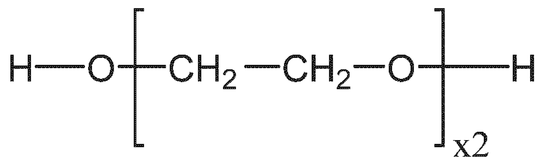

(AG-2),

x2 für eine ganze Zahl von 101 bis 1000, bevorzugt für eine ganze Zahl von 105 bis 800, weiter bevorzugt für eine ganze Zahl von 107 bis 600, noch weiter bevorzugt für eine ganze Zahl von 109 bis 400 und ganz besonders bevorzugt für eine ganze Zahl von 110 bis 200 steht.

**5.** Mittel nach einem der Ansprüche 1 bis 4, **dadurch gekennzeichnet, dass** es
(a1) mindestens Alkylenglycol der Formel (AG-3) enthält,

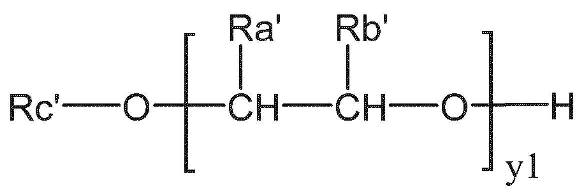

(AG-3),

wobei

y1 für eine ganze Zahl von 1 bis 1000, bevorzugt für eine ganze Zahl von 3 bis 100, besonders bevorzugt für eine ganze Zahl von 5 bis 50, steht,
Ra', Rb' unabhängig voneinander für ein Wasserstoffatom oder für eine $C_1$-$C_6$-Alkylgruppe stehen, wobei bevorzugt einer der Reste aus Ra' und Rb' für ein Wasserstoffatom steht und der verbleibende der beiden Reste für eine $C_1$-$C_6$-Alkylgruppe, bevorzugt für eine Methylgruppe, steht, und
Rc' für eine $C_1$-$C_6$-Alkylgruppe steht.

**6.** Mittel nach einem der Ansprüche 1 bis 5, **dadurch gekennzeichnet, dass** es - bezogen auf das Gesamtgewicht des

Mittels - ein oder mehrere Alkylenglycol der Formel (AG) (a1) in einer Gesamtmenge von 10,0 bis 99,0 Gew.-%, bevorzugt von 30,0 bis 99,0 Gew.-%, weiter bevorzugt von 50,0 bis 99,0 Gew.-% und ganz besonders bevorzugt von 70,0 bis 99,0 Gew.-% enthält.

**7.** Mittel nach einem der Ansprüche 1 bis 6, **dadurch gekennzeichnet, dass** es - bezogen auf das Gesamtgewicht des Mittels - (a1) ein oder mehrere Alkylenglycole der Formel (AG-1) in einer Gesamtmenge von 20,0 bis 99,0 Gew.-%, bevorzugt von 40,0 bis 95,0 Gew.-%, besonders bevorzugt von 60,0 bis 90,0 Gew.-%, enthält und/oder es ein oder mehrere Alkylenglycole der Formel (AG-2) in einer Gesamtmenge von 1,0 bis 35,0 Gew.-%, bevorzugt von 3,0 bis 30,0 Gew.-%, besonders bevorzugt von 4,0 bis 25,0 Gew.-%, enthält und/oder es ein oder mehrere Alkylenglycole der Formel (AG-3) in einer Gesamtmenge von 0,1 bis 20,0 Gew-%, bevorzugt von 0,2 bis 10,0 Gew.-%, besonders bevorzugt von 0,4 bis 5,0 Gew.-% enthält.

**8.** Mittel nach einem der Ansprüche 1 bis 7, **dadurch gekennzeichnet, dass** es mindestens ein anorganisches Pigment (a2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

**9.** Mittel nach einem der Ansprüche 1 bis 8, **dadurch gekennzeichnet, dass** es mindestens ein organisches Pigment (a2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

**10.** Mittel nach einem der Ansprüche 1 bis 9, **dadurch gekennzeichnet, dass** es - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

**11.** Mittel nach einem der Ansprüche 1 bis 10, **dadurch gekennzeichnet, dass** es (a3) mindestens ein aminofunktionalisiertes Silikonpolymer mit mindestens einer sekundären Aminogruppe enthält.

**12.** Mittel nach einem der Ansprüche 1 bis 11, **dadurch gekennzeichnet, dass** es mindestens ein aminofunktionalisiertes Silikonpolymer (a3) enthält, das mindestens eine Struktureinheit der Formel (Si-Amino) umfasst,

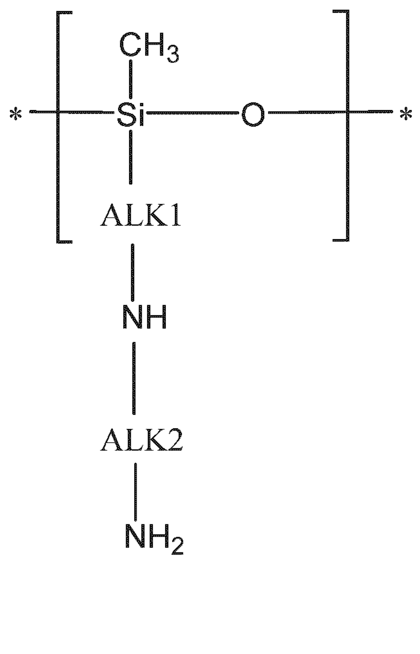

(Si-Amino)

wobei

ALK1 und ALK2 unabhängig voneinander für eine lineare oder verzweigte, zweiwertige $C_1$-$C_{20}$-Alkylengruppe stehen.

13. Mittel nach einem der Ansprüche 1 bis 12, **dadurch gekennzeichnet, dass** es mindestens ein aminofunktionalisiertes Silikonpolymer (a3) enthält, das Struktureinheiten der Formel (Si-I) und der Formel (Si-II) umfasst

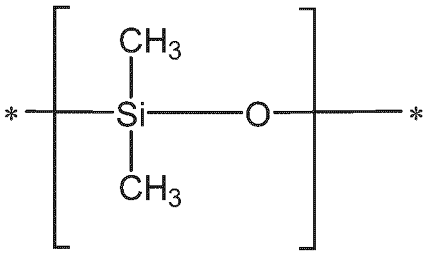

(Si-I)

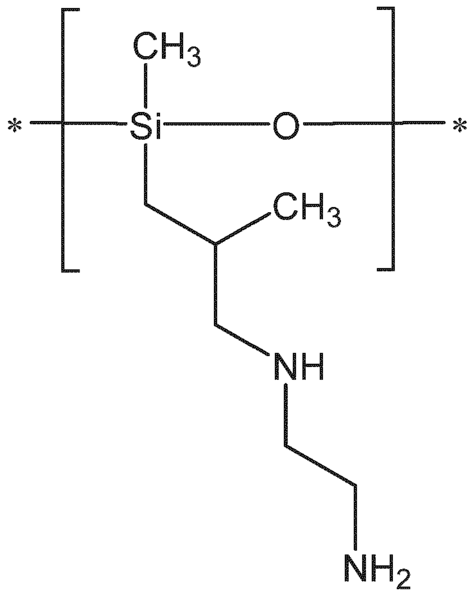

(Si-II).

14. Mittel nach einem der Ansprüche 1 bis 13, **dadurch gekennzeichnet, dass** es - bezogen auf das Gesamtgewicht des Mittels - ein oder mehrere aminofunktionalisierte Silikonpolymere (a3) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

15. Mittel nach einem der Ansprüche 1 bis 14, **dadurch gekennzeichnet, dass** es
(a4) mindestens ein Anlagerungsprodukt von $C_1$-$C_6$-Alkylenoxid(en) an einen Ester enthält, der durch Veresterung von einer $C_{12}$-$C_{24}$-Fettsäure mit einem aromatischen $C_1$-$C_{12}$-Alkohol aus der Gruppe aus Benzylalkohol, Phenol, 2-Phenylethylalkohol und 2-Phenoxyethanol, ganz besonders bevorzugt Benzylalkohol, erhalten wird.

16. Mittel nach einem der Ansprüche 1 bis 15, **dadurch gekennzeichnet, dass** es - bezogen auf das Gesamtgewicht des Mittels - ein oder mehrere alkoxylierte Fettsäureester (a4) in einer Gesamtmenge von 0,1 bis 20,0 Gew.-%, bevorzugt 0,5 bis 15,0 Gew.-%, weiter bevorzugt von 1,0 bis 10,0 Gew.-%, noch weiter bevorzugt von 1,0 bis 8,0 Gew.-% und ganz besonders bevorzugt von 1,0 bis 5,0 Gew.-% enthält.

17. Verfahren zum Färben von keratinischem Material, insbesondere menschlichen Haaren, bei welchem ein Mittel, wie es in den Ansprüchen 1 bis 16 beschrieben ist, auf die keratinischen Fasern aufgetragen wird und gegebenenfalls nach einer Einwirkzeit von 30 Sekunden bis 45 Minuten wieder ausgespült wird.

## Claims

1. A water-free agent for dyeing keratinous material, in particular human hair, containing

(a1) at least alkylene glycol of formula (AG)

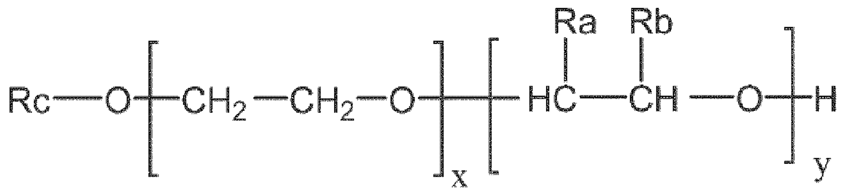

(AG),

where

x represents an integer from 0 to 10,000,
y represents an integer from 0 to 10,000,
Ra, Rb represent, independently of one another, a hydrogen atom, a $C_1$-$C_6$ alkyl group or a hydroxy-$C_1$-$C_6$ alkyl group,
Rc represents a hydrogen atom, a $C_1$-$C_6$ alkyl group, a phenyl group or a benzyl group,
with the proviso that x + y is at least 1, and

(a2) at least one pigment, and
(a3) at least one amino-functionalized silicone polymer, and
(a4) at least one addition product of $C_1$-$C_6$ alkylene oxide(s) to the esters of $C_{12}$-$C_{30}$ fatty acids and aromatic $C_1$-$C_{12}$ alcohols.

2. The agent according to claim 1, **characterized in that** it contains
(a1) at least alkylene glycol of formula (AG), where

x represents an integer from 1 to 10,000, and
y represents the number 0, and
Rc represents a hydrogen atom.

3. The agent according to one of claims 1 to 2, **characterized in that** it contains
(a1) at least one alkylene glycol of formula (AG-1), where

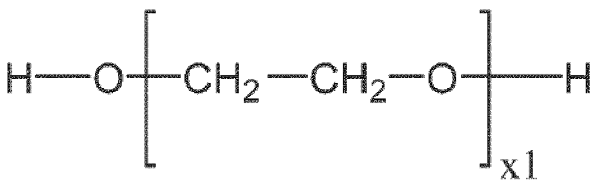

(AG-1),

x1 represents an integer from 1 to 100, preferably an integer from 1 to 80, more preferably an integer from 2 to 60, even more preferably an integer from 3 to 40, even more preferably an integer from 4 to 20, and very particularly preferably an integer from 6 to 15.

4. The agent according to one of claims 1 to 3, **characterized in that** it contains
(a1) at least one alkylene glycol of formula (AG-2), where

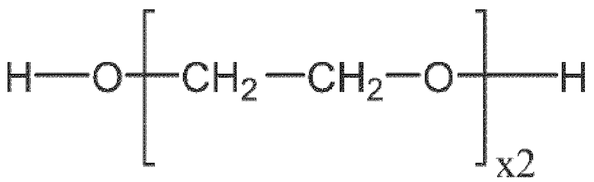

(AG-2),

x2 represents an integer from 101 to 1,000, preferably an integer from 105 to 800, more preferably an integer from 107 to 600, even more preferably an integer from 109 to 400, and very particularly preferably an integer from 110 to 200.

**5.** The agent according to one of claims 1 to 4, **characterized in that** it contains (a1) at least alkylene glycol of formula (AG-3),

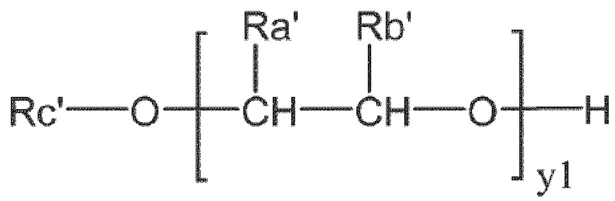

(AG-3),

where

y1 represents an integer from 1 to 1,000, preferably an integer from 3 to 100, particularly preferably an integer from 5 to 50,
Ra', Rb' represent, independently of one another, a hydrogen atom or a $C_1$-$C_6$ alkyl group, wherein preferably one of the functional groups of Ra' and Rb' represents a hydrogen atom, and the remaining one of the two functional groups represents a $C_1$-$C_6$ alkyl group, preferably a methyl group, and
Rc' represents a $C_1$-$C_6$ alkyl group.

**6.** The agent according to one of claims 1 to 5, **characterized in that** it contains, based on the total weight of the agent, one or more alkylene glycols of formula (AG) (a1) in a total amount from 10.0 to 99.0 wt.%, preferably from 30.0 to 99.0 wt.%, more preferably from 50.0 to 99.0 wt.%, and very particularly preferably from 70.0 to 99.0 wt.%.

**7.** The agent according to one of claims 1 to 6, **characterized in that** it contains, based on the total weight of the agent, (a1) one or more alkylene glycols of formula (AG-1) in a total amount from 20.0 to 99.0 wt.%, preferably from 40.0 to 95.0 wt.%, more preferably from 60.0 to 90.0 wt.%, and/or it contains one or more alkylene glycols of formula (AG-2) in a total amount from 1.0 to 35.0 wt.%, preferably from 3.0 to 30.0 wt.%, particularly preferably from 4.0 to 25.0 wt.%, and/or it contains one or more alkylene glycols of formula (AG-3) in a total amount from 0.1 to 20.0 wt.%, preferably from 0.2 to 10.0 wt.%, particularly preferably from 0.4 to 5.0 wt.%.

**8.** The agent according to one of claims 1 to 7, **characterized in that** it contains at least one inorganic pigment (a2), which is preferably selected from the group of colored metal oxides, metal hydroxides, metal oxide hydrates, silicates, metal sulfides, complex metal cyanides, metal sulfates, bronze pigments, and/or from mica-based colored pigments which are coated with at least one metal oxide and/or a metal oxychloride.

**9.** The agent according to one of claims 1 to 8, **characterized in that** it contains at least one organic pigment (a2) which is preferably selected from the group of carmine, quinacridone, phthalocyanine, sorghum, blue pigments having the Color Index numbers CI 42090, CI 69800, CI 69825, CI 73000, CI 74100 or CI 74160, yellow pigments having the Color Index numbers CI 11680, CI 11710, CI 15985, CI 19140, CI 20040, CI 21100, CI 21108, CI 47000 or CI 47005, green pigments having the Color Index numbers CI 61565, CI 61570 or CI 74260, orange pigments having the Color Index numbers CI 11725, CI 15510, CI 45370 or CI 71105, red pigments having the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

**10.** The agent according to one of claims 1 to 9, **characterized in that** it contains, based on the total weight of the agent, one or more pigments (a2) in a total amount from 0.01 to 10.0 wt.%, preferably 0.1 to 5.0 wt.%, more preferably from 0.2 to 2.5 wt.%, and very particularly preferably from 0.25 to 1.5 wt.%.

**11.** The agent according to one of claims 1 to 10, **characterized in that** it contains (a3) at least one amino-functionalized silicone polymer with at least one secondary amino group.

**12.** The agent according to one of claims 1 to 11, **characterized in that** it contains at least one amino-functionalized silicone polymer (a3) which comprises at least one structural unit of formula (Si-amino),

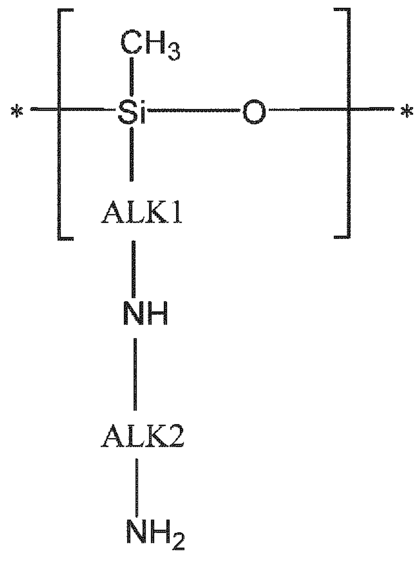

(Si-amino)

where
ALK1 and ALK2 represent, independently of one another, a linear or branched, divalent $C_1$-$C_{20}$ alkylene group.

**13.** The agent according to one of claims 1 to 12, **characterized in that** it contains at least one amino-functionalized silicone polymer (a3) which comprises structural units of formula (Si-I) and formula (Si-II)

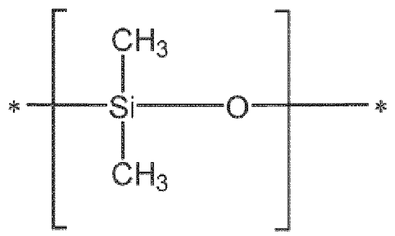

(Si-I)

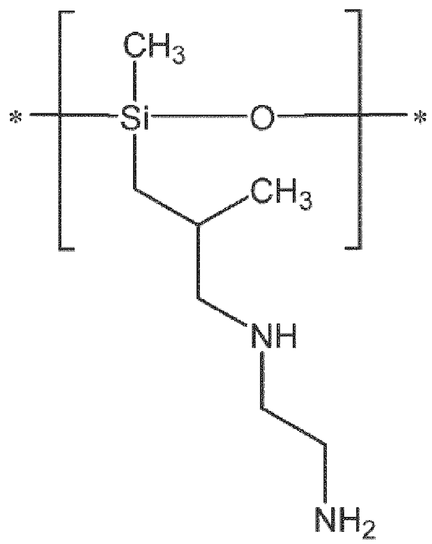

(Si-II).

**14.** The agent according to one of claims 1 to 13, **characterized in that** it contains, based on the total weight of the agent, one or more amino-functionalized silicone polymers (a3) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

**15.** The agent according to one of claims 1 to 14, **characterized in that** it contains
(a4) at least one addition product of $C_1$-$C_6$ alkylene oxide(s) to an ester which is obtained by esterification of a $C_{12}$-$C_{24}$ fatty acid with an aromatic $C_1$-$C_{12}$ alcohol from the group of benzyl alcohol, phenol, 2-phenylethyl alcohol and 2-phenoxyethanol, very particularly preferably benzyl alcohol.

**16.** The agent according to one of claims 1 to 15, **characterized in that** it contains, based on the total weight of the agent, one or more alkoxylated fatty acid esters (a4) in a total amount from 0.1 to 20.0 wt.%, preferably 0.5 to 15.0 wt.%, more preferably from 1.0 to 10.0 wt.%, even more preferably from 1.0 to 8.0 wt.%, and very particularly preferably from 1.0 to 5.0 wt.%.

17. A method for dyeing keratinous material, in particular human hair, in which an agent as described in claims 1 to 16 is applied to the keratinous fibers and optionally rinsed after an exposure time of 30 seconds to 45 minutes.

## Revendications

1. Agent anhydre permettant la coloration d'une matière kératinique, en particulier de cheveux humains, contenant

   (a1) au moins un alkylène glycol de formule (AG)

   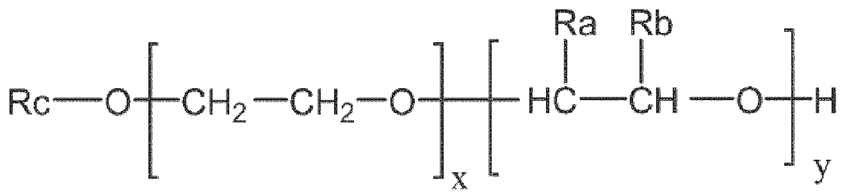

   (AG),

   où

   x représente un nombre entier allant de 0 à 10 000,
   y représente un nombre entier allant de 0 à 10 000,
   Ra, Rb représentent, indépendamment l'un de l'autre, un atome d'hydrogène, un groupe alkyle en $C_1$-$C_6$ ou un groupe hydroxy alkyle en $C_1$-$C_6$,
   Rc représente un atome d'hydrogène, un groupe alkyle en $C_1$-$C_6$, un groupe phényle ou un groupe benzyle, à condition que x + y soit au moins égal à 1, et

   (a2) au moins un pigment, et
   (a3) au moins un polymère de silicone aminofonctionnalisé, et
   (a4) au moins un produit d'addition d'oxyde(s) d'alkylène en $C_1$-$C_6$ aux esters d'acides gras en $C_{12}$-$C_{30}$ et d'alcools aromatiques en $C_1$-$C_{12}$.

2. Agent selon la revendication 1, **caractérisé en ce qu'**il
   (a1) contient au moins un alkylène glycol de formule (AG), où

   x représente un nombre entier allant de 1 à 10 000, et
   y représente le nombre 0, et
   Rc représente un atome d'hydrogène.

3. Agent selon l'une des revendications 1 à 2, **caractérisé en ce qu'**il
   (a1) contient au moins un alkylène glycol de formule (AG-1), où

   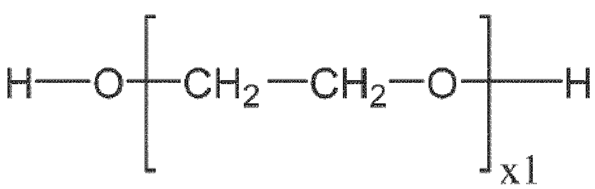

   (AG-1),

   x1 représente un nombre entier allant de 1 à 100, de préférence un nombre entier allant de 1 à 80, plus préférablement un nombre entier allant de 2 à 60, encore plus préférablement un nombre entier allant de 3 à 40, encore plus préférablement un nombre entier allant de 4 à 20 et de manière tout particulièrement préférée un nombre entier allant de 6 à 15.

4. Agent selon l'une des revendications 1 à 3, **caractérisé en ce qu'**il
   (a1) contient au moins un alkylène glycol de formule (AG-2), où

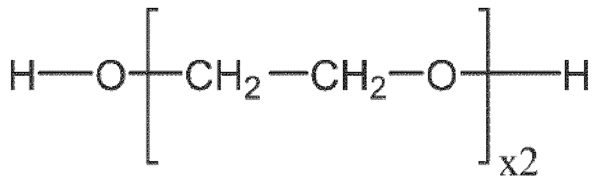

(AG-2),

x2 représente un nombre entier allant de 101 à 1 000, de préférence un nombre entier allant de 105 à 800, plus préférablement un nombre entier allant de 107 à 600, encore plus préférablement un nombre entier allant de 109 à 400 et de manière tout particulièrement préférée un nombre entier allant de 110 à 200.

5. Agent selon l'une des revendications 1 à 4, **caractérisé en ce qu'**il
(a1) contient au moins un alkylène glycol de formule (AG-3),

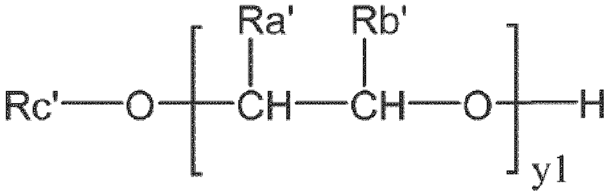

(AG-3),

où

y1 représente un nombre entier allant de 1 à 1 000, de préférence un nombre entier allant de 3 à 100, de manière particulièrement préférée un nombre entier allant de 5 à 50,
Ra', Rb' représentent, indépendamment l'un de l'autre, un atome d'hydrogène ou un groupe alkyle en $C_1$-$C_6$, où, de préférence, l'un des radicaux parmi Ra' et Rb' représente un atome d'hydrogène et le radical restant parmi les deux radicaux représente un groupe alkyle en $C_1$-$C_6$, de préférence un groupe méthyle, et
Rc' représente un groupe alkyle en $C_1$-$C_6$.

6. Agent selon l'une des revendications 1 à 5, **caractérisé en ce qu'**il contient, par rapport au poids total de l'agent, un ou plusieurs alkylènes glycols de formule (AG) (a1) en une quantité totale allant de 10,0 à 99,0 % en poids, de préférence de 30,0 à 99,0 % en poids, plus préférablement de 50,0 à 99,0 % en poids et de manière tout particulièrement préférée de 70,0 à 99,0 % en poids.

7. Agent selon l'une des revendications 1 à 6, **caractérisé en ce qu'**il contient, par rapport au poids total de l'agent, (a1) un ou plusieurs alkylènes glycols de formule (AG-1) en une quantité totale allant de 20,0 à 99,0 % en poids, de préférence de 40,0 à 95,0 % en poids, de manière particulièrement préférée de 60,0 à 90,0 % en poids, et/ou il contient un ou plusieurs alkylènes glycols de formule (AG-2) en une quantité totale allant de 1,0 à 35,0 % en poids, de préférence de 3,0 à 30,0 % en poids, de manière particulièrement préférée de 4,0 à 25,0 % en poids, et/ou il contient un ou plusieurs alkylènes glycols de formule (AG-3) en une quantité totale allant de 0,1 à 20,0 % en poids, de préférence de 0,2 à 10,0 % en poids, de manière particulièrement préférée de 0,4 à 5,0 % en poids.

8. Agent selon l'une des revendications 1 à 7, **caractérisé en ce qu'**il contient au moins un pigment inorganique (a2) qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica qui sont recouverts d'au moins un oxyde métallique et/ou oxychlorure métallique.

9. Agent selon l'une des revendications 1 à 8, **caractérisé en ce qu'**il contient au moins un pigment organique (a2) qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI

15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

**10.** Agent selon l'une des revendications 1 à 9, **caractérisé en ce qu'**il contient, par rapport au poids total de l'agent, un ou plusieurs pigments (a2) en une quantité totale allant de 0,01 à 10,0 % en poids, de préférence de 0,1 à 5,0 % en poids, plus préférablement de 0,2 à 2,5 % en poids et de manière tout particulièrement préférée de 0,25 à 1,5 % en poids.

**11.** Agent selon l'une des revendications 1 à 10, **caractérisé en ce qu'**il
(a3) contient au moins un polymère de silicone aminofonctionnalisé comportant au moins un groupe amine secondaire.

**12.** Agent selon l'une des revendications 1 à 11, **caractérisé en ce qu'**il contient au moins un polymère de silicone aminofonctionnalisé (a3) qui comprend au moins un motif structural de formule (Si-Amino),

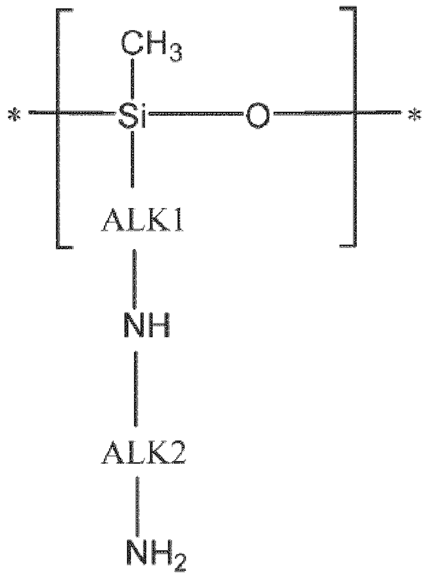

(Si-Amino)

où
ALK1 et ALK2 représentent, indépendamment l'un de l'autre, un groupe alkylène en $C_1$-$C_{20}$ bivalent, linéaire ou ramifié.

**13.** Agent selon l'une des revendications 1 à 12, **caractérisé en ce qu'**il contient au moins un polymère de silicone aminofonctionnalisé (a3) qui comprend des motifs structuraux de formule (Si-I) et de formule (Si-II)

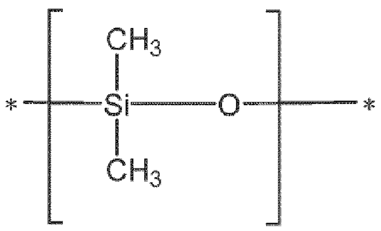

(Si-I)

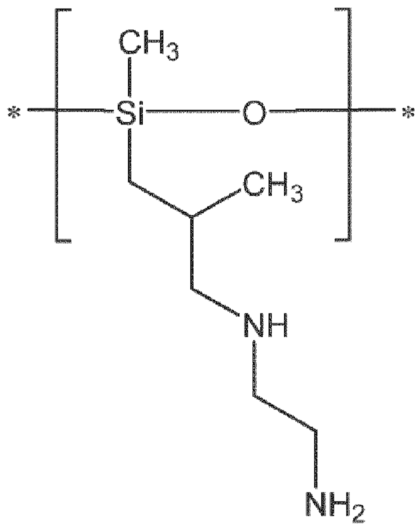

(Si-II).

**14.** Agent selon l'une des revendications 1 à 13, **caractérisé en ce qu'**il contient, par rapport au poids total de l'agent, un ou plusieurs polymères de silicone aminofonctionnalisés (a3) en une quantité totale allant de 0,1 à 8,0 % en poids, de préférence de 0,2 à 5,0 % en poids, plus préférablement de 0,3 à 3,0 % en poids et de manière tout particulièrement préférée de 0,4 à 2,5 % en poids.

**15.** Agent selon l'une des revendications 1 à 14, **caractérisé en ce qu'**il
(a4) contient au moins un produit d'addition d'oxyde(s) d'alkylène en $C_1$-$C_6$ à un ester obtenu par estérification d'un acide gras en $C_{12}$-$C_{24}$ avec un alcool aromatique en $C_1$-$C_{12}$ du groupe constitué d'alcool benzylique, phénol, alcool 2-phényléthylique et 2-phénoxyéthanol, de manière tout particulièrement préférée d'alcool benzylique.

**16.** Agent selon l'une des revendications 1 à 15, **caractérisé en ce qu'**il contient, par rapport au poids total de l'agent, un ou plusieurs esters d'acides gras alcooxylés (a4) en une quantité totale allant de 0,1 à 20,0 % en poids, de préférence de 0,5 à 15,0 % en poids, plus préférablement de 1,0 à 10,0 % en poids, encore plus préférablement de 1,0 à 8,0 % en poids et de manière tout particulièrement préférée de 1,0 à 5,0 % en poids.

**17.** Procédé permettant la coloration d'une matière kératinique, en particulier de cheveux humains, dans lequel un agent tel que décrit dans les revendications 1 à 16 est appliqué sur les fibres kératiniques et est éventuellement rincé après un temps d'action allant de 30 secondes à 45 minutes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2020260097 A1

### In der Beschreibung aufgeführte Nicht-Patentliteratur

- *CHEMICAL ABSTRACTS*, 25322-68-3
- *CHEMICAL ABSTRACTS*, 9003-13-8
- *CHEMICAL ABSTRACTS*, 106842-44-8
- *CHEMICAL ABSTRACTS*, 75-21-8
- *CHEMICAL ABSTRACTS*, 75-56-9
- *CHEMICAL ABSTRACTS*, 642443-86-5